# EUROPEAN PATENT APPLICATION

(11) **EP 1 482 047 A1**
(43) Date of publication of application: **01.12.2004**
(21) Application number: 03743544.3
(22) Date of filing: 28.02.2003
(51) Int. Cl.: C12N 15/29, C12N 15/82, C12N 9/90, C12Q 1/68

(54) **PROTEIN OR POLYPEPTIDE HAVING LACHRYMATOR SYNTHASE ACTIVITY, DNA ENCODING THE PROTEIN OR THE POLYPEPTIDE, PROCESS FOR PRODUCING PROTEIN OR POLYPEPTIDE HAVING LACHRYMATOR SYNTHASE ACTIVITY USING THE DNA, AND NUCLEIC ACID MOLECULE INHIBITING THE TRANSLATION OF mRNA CONCERNING THE PROTEIN OR THE PEPTID**

(30) Priority: 01.03.2002 JP 2002056523; 01.03.2002 JP 2002056558; 20.09.2002 JP 2002275799
(71) Applicant: House Foods Corporation, Higashi-Osaka-shi, Osaka 577-0036 (JP)
(72) Inventor: IMAI, Shinsuke, HOUSE FOODS CORPORATION, Higashi-Osaka-Shi, Osaka 577-0036 (JP); TSUGE, Nobuaki, HOUSE FOODS CORPORATION, Higashi-Osaka-Shi, Osaka 577-0036 (JP); KAMATA, Yasuhiro, HOUSE FOODS CORPORATION, Higashi-Osaka-Shi, Osaka 577-0036 (JP); MASAMURA, Noriya, HOUSE FOODS CORPORATION, Higashi-Osaka-Shi, Osaka 577-0036 (JP); SHONO, Jinji, HOUSE FOODS CORPORATION, Higashi-Osaka-Shi, Osaka 577-0036 (JP)
(74) Representative: Hallybone, Huw George
(86) International application number: PCT/JP2003/002396
(87) International publication number: WO 2003/074706

(57) **Abstract**

The present invention relates to protein or polypeptide capable of converting 1-propenylsulfenic acid concerning the formation of lachrymatory factors generated when plants such as onions are broken into pieces or cut into lachrymatory factors, and also to DNA (lachrymatory factor-producing enzyme genes) encoding the protein or polypeptide; a primer usable for isolating a gene of a lachrymatory factor-producing enzyme of the genus allium and a method for isolating the gene with the primer; a recombinant vector containing the DNA; a transformant containing the recombinant vector containing the DNA; a method for preparing protein or a polypeptide having lachrymatory factor-producing enzymatic activity by culturing host cells transformed with the recombinant vector containing the DNA; protein or a polypeptide having the lachrymatory factor-producing enzymatic activity; and a nucleic acid molecule having a function of repressing the translation of mRNA on protein or polypeptide having the lachrymatory factor-producing enzymatic activity.

SEQUENCE LISTING

<110>  House Foods Corporation

<120>  Protein or polypeptide having lacrimator generation enzyme activity, DNA
coding for said protein or polypeptide, method for preparation of the protein or
polypeptide having lacrimator generation enzyme activity by using said DNA and
nucleic acid having an effect of inhibiting translation of mRNA with respect to said
protein or polypeptide

<130>  Y1K0092

<150>  JP 2002-56523
<151>  2002-03-01

<150>  JP 2002-56558
<151>  2002-03-01

<150>  JP 2002-275799
<151>  2002-09-20

<160>  30

<170>  PatentIn version 3.1

<210>  1
<211>  739
<212>  DNA
<213>  Allium fistulosum L.

<220>
<221>  CDS
<222>  (58)..(564)
<223>  P

<220>
<221>  polyA_site
<222>  (723)..(739)
<223>  P

<400>  1
cacaaattca aaactcacat ttcgttaaat ttagaagaat tattcaatcg ggaaaaa        57

atg gag cta aat cct ggt gcg cct gct gta gtc act gat ggt gct aac      105
Met Glu Leu Asn Pro Gly Ala Pro Ala Val Val Thr Asp Gly Ala Asn
1               5                   10                  15

gga gct cga aaa tgg agc ggc aaa gtc cat gct ttg ctt cca aat tca      153
Gly Ala Arg Lys Trp Ser Gly Lys Val His Ala Leu Leu Pro Asn Ser
            20                  25                  30

aag cca gag caa gca tgg agg cta cta aag gac ttt att aac ctt cac      201
Lys Pro Glu Gln Ala Trp Arg Leu Leu Lys Asp Phe Ile Asn Leu His
        35                  40                  45

aag atc atg cct tcg ttg tca gtc tgt gaa ctg gta gaa ggt aag gcc      249
Lys Ile Met Pro Ser Leu Ser Val Cys Glu Leu Val Glu Gly Lys Ala
    50                  55                  60

aat gtt gtt ggt tgt gtt cgc cac gtt aaa ggt ata atg cac cca ata      297
Asn Val Val Gly Cys Val Arg His Val Lys Gly Ile Met His Pro Ile
65                  70                  75                  80

gaa gag gaa ttt tgg gcc aag gag aag ctg gtg gca ctg gat aat aag      345
Glu Glu Glu Phe Trp Ala Lys Glu Lys Leu Val Ala Leu Asp Asn Lys
                85                  90                  95

aac atg agc tac agt tat att ttt act gag tgt ttt aca ggg ttc gag      393

Asn Met Ser Tyr Ser Tyr Ile Phe Thr Glu Cys Phe Thr Gly Phe Glu
            100                 105                 110

gat tac acg gct acc atg caa ata gtg gag gga cct gag cac aag gga      441
Asp Tyr Thr Ala Thr Met Gln Ile Val Glu Gly Pro Glu His Lys Gly
        115                 120                 125

tgt aga ttt gac tgg tct ttt cag tgc aag tat atc gag ggt atg act      489
Cys Arg Phe Asp Trp Ser Phe Gln Cys Lys Tyr Ile Glu Gly Met Thr
    130                 135                 140

gaa tct gca ttc gcc gag att ctg cag cat tgg gct act gaa att ggt      537
Glu Ser Ala Phe Ala Glu Ile Leu Gln His Trp Ala Thr Glu Ile Gly
145                 150                 155                 160

cag aaa atc gaa gag att tgc aat gct tgattatgaa tatcggttat            584
Gln Lys Ile Glu Glu Ile Cys Asn Ala
                165

ggtttggtgc attgtgtgtg ttttaaaccg tatcttgtga tgtaataaag taacgtaata    644

tgtgcacgta ataagtaagc ctgagtgttg tgtgttcaat aaaaaagaac ttgctttttg    704

catgttctaa tgcttttcaa aaaaaaaaaa aaaaa                               739

<210>  2
<211>  169
<212>  PRT
<213>  Allium fistulosum L.

<400>  2

Met Glu Leu Asn Pro Gly Ala Pro Ala Val Val Thr Asp Gly Ala Asn
1               5                   10                  15

Gly Ala Arg Lys Trp Ser Gly Lys Val His Ala Leu Leu Pro Asn Ser
            20                  25                  30

Lys Pro Glu Gln Ala Trp Arg Leu Leu Lys Asp Phe Ile Asn Leu His
        35                  40                  45

Lys Ile Met Pro Ser Leu Ser Val Cys Glu Leu Val Glu Gly Lys Ala
    50                  55                  60

Asn Val Val Gly Cys Val Arg His Val Lys Gly Ile Met His Pro Ile
65                  70                  75                  80

Glu Glu Glu Phe Trp Ala Lys Glu Lys Leu Val Ala Leu Asp Asn Lys
                85                  90                  95

Asn Met Ser Tyr Ser Tyr Ile Phe Thr Glu Cys Phe Thr Gly Phe Glu
            100                 105                 110

Asp Tyr Thr Ala Thr Met Gln Ile Val Glu Gly Pro Glu His Lys Gly
        115                 120                 125

Cys Arg Phe Asp Trp Ser Phe Gln Cys Lys Tyr Ile Glu Gly Met Thr
    130                 135                 140

Glu Ser Ala Phe Ala Glu Ile Leu Gln His Trp Ala Thr Glu Ile Gly
145                 150                 155                 160

Gln Lys Ile Glu Glu Ile Cys Asn Ala
                165

<210>  3
<211>  682
<212>  DNA
<213>  Allium fistulosum L.

<220>
<221>  CDS
<222>  (1)..(507)
<223>  P

<220>
<221>  polyA_site
<222>  (666)..(682)
<223>  P

<400>  3
atg gag cta aat cct ggt gcg cct gct gta gtc act gat ggt gct aac        48
Met Glu Leu Asn Pro Gly Ala Pro Ala Val Val Thr Asp Gly Ala Asn
1               5                   10                  15

gga gct cga aaa tgg agc ggc aaa gtc cat gct ttg ctt cca aat tca        96
Gly Ala Arg Lys Trp Ser Gly Lys Val His Ala Leu Leu Pro Asn Ser
            20                  25                  30

aag cca gag caa gca tgg agg cta cta aag gac ttt att aac ctt cac       144
Lys Pro Glu Gln Ala Trp Arg Leu Leu Lys Asp Phe Ile Asn Leu His
        35                  40                  45

aag atc atg cct tcg ttg tca gtc tgt gaa ctg gta gaa ggt gag gcc       192

Lys Ile Met Pro Ser Leu Ser Val Cys Glu Leu Val Glu Gly Glu Ala
    50                  55                  60

aat gtt gtt ggt tgt gtt cgc cac gtt aaa ggt ata atg cac cca ata 240
Asn Val Val Gly Cys Val Arg His Val Lys Gly Ile Met His Pro Ile
65                  70                  75                  80

gaa gag gaa ttt tgg gcc aag gag aag ctg gtg gca ctg gat aat aag 288
Glu Glu Glu Phe Trp Ala Lys Glu Lys Leu Val Ala Leu Asp Asn Lys
                85                  90                  95

aac atg agc tac agt tat att ttt act gag tgt ttt aca ggg ttc gag 336
Asn Met Ser Tyr Ser Tyr Ile Phe Thr Glu Cys Phe Thr Gly Phe Glu
            100                 105                 110

gat tac acg gct acc atg caa ata gtg gag gga cct gag cac aag gga 384
Asp Tyr Thr Ala Thr Met Gln Ile Val Glu Gly Pro Glu His Lys Gly
        115                 120                 125

tgt aga ttt gac tgg tct ttt cag tgc aag tat atc gag ggt atg act 432
Cys Arg Phe Asp Trp Ser Phe Gln Cys Lys Tyr Ile Glu Gly Met Thr
    130                 135                 140

gaa tct gca ttc gcc gag att ctg cag cat tgg gct act gaa att ggt 480
Glu Ser Ala Phe Ala Glu Ile Leu Gln His Trp Ala Thr Glu Ile Gly
145                 150                 155                 160

cag aaa atc gaa gag att tgc aat gct tgattatgaa tatcggttat 527
Gln Lys Ile Glu Glu Ile Cys Asn Ala
                165

ggtttggtgc attgtgtgtg ttttaaaccg tatcttgtga tgtaataaag taacgtaata 587

tgtgcacgta ataagtaagc ctgagtgttg tgtgttcaat aaaaaagaac ttgcttttg 647

catgttctaa tgcttttcaa aaaaaaaaaa aaaaa 682

<210> 4
<211> 169
<212> PRT
<213> Allium fistulosum L.

<400> 4

Met Glu Leu Asn Pro Gly Ala Pro Ala Val Val Thr Asp Gly Ala Asn
1               5                   10                  15

Gly Ala Arg Lys Trp Ser Gly Lys Val His Ala Leu Leu Pro Asn Ser
            20                  25                  30

Lys Pro Glu Gln Ala Trp Arg Leu Leu Lys Asp Phe Ile Asn Leu His
        35                  40                  45

Lys Ile Met Pro Ser Leu Ser Val Cys Glu Leu Val Glu Gly Glu Ala
    50                  55                  60

Asn Val Val Gly Cys Val Arg His Val Lys Gly Ile Met His Pro Ile
65                  70                  75                  80

Glu Glu Glu Phe Trp Ala Lys Glu Lys Leu Val Ala Leu Asp Asn Lys
                85                  90                  95

Asn Met Ser Tyr Ser Tyr Ile Phe Thr Glu Cys Phe Thr Gly Phe Glu
            100                 105                 110

Asp Tyr Thr Ala Thr Met Gln Ile Val Glu Gly Pro Glu His Lys Gly
        115                 120                 125

Cys Arg Phe Asp Trp Ser Phe Gln Cys Lys Tyr Ile Glu Gly Met Thr
    130                 135                 140

Glu Ser Ala Phe Ala Glu Ile Leu Gln His Trp Ala Thr Glu Ile Gly
145                 150                 155                 160

Gln Lys Ile Glu Glu Ile Cys Asn Ala
                165

<210>  5
<211>  730
<212>  DNA
<213>  Allium chinense L.

<220>
<221>  CDS
<222>  (54)..(560)
<223>  P

<220>
<221>  polyA_site
<222>  (715)..(730)
<223>  P

<400>  5
cacaattcaa actcactttt cgttatattt agaagattac ccaatcagaa aaa atg        56

Met
1

gag caa aat tct ggt acg ctt gct gta gtc act gat ggt gct aaa gga 104
Glu Gln Asn Ser Gly Thr Leu Ala Val Val Thr Asp Gly Ala Lys Gly
            5                   10                  15

gct gca aaa tgg aga ggc aaa gtc cat gct ttg ctt cca aat aca aaa 152
Ala Ala Lys Trp Arg Gly Lys Val His Ala Leu Leu Pro Asn Thr Lys
        20                  25                  30

cca gag caa gca tgg aca cta cta aaa gac ttt att aac ctt cac aag 200
Pro Glu Gln Ala Trp Thr Leu Leu Lys Asp Phe Ile Asn Leu His Lys
    35                  40                  45

atc atg cct tcg ttg tca gtt tgt gaa ctg gtt gaa ggc gag gcc aat 248
Ile Met Pro Ser Leu Ser Val Cys Glu Leu Val Glu Gly Glu Ala Asn
50                  55                  60                  65

gtt gtt ggt tgt gtt cgc cac gtt aaa ggt ata atg cac cca atg gaa 296
Val Val Gly Cys Val Arg His Val Lys Gly Ile Met His Pro Met Glu
                70                  75                  80

gag gaa ttt tgg gcc aag gag aag ctg gtt gca ctg gat gat aag aac 344
Glu Glu Phe Trp Ala Lys Glu Lys Leu Val Ala Leu Asp Asp Lys Asn
            85                  90                  95

atg agc tgt agt tat att ttt gtt gag tgt ttt aca ggg tac gag gat 392
Met Ser Cys Ser Tyr Ile Phe Val Glu Cys Phe Thr Gly Tyr Glu Asp
        100                 105                 110

tac aca gct acc atg caa ata gtg gag gga tct gag cac aag gga tgt 440
Tyr Thr Ala Thr Met Gln Ile Val Glu Gly Ser Glu His Lys Gly Cys
    115                 120                 125

aga ttt gac tgg tct ttt cag tgt aag tat atc gag ggt atg act gaa 488

Arg Phe Asp Trp Ser Phe Gln Cys Lys Tyr Ile Glu Gly Met Thr Glu
130                 135                 140                 145

tct gca ttc acc gat gtt ctg cag cat tgg gct act gag att ggt cag      536
Ser Ala Phe Thr Asp Val Leu Gln His Trp Ala Thr Glu Ile Gly Gln
                150                 155                 160

aaa att gaa gag att tgc aat gct tgatcatgaa taccggttat gttgtgatgc      590
Lys Ile Glu Glu Ile Cys Asn Ala
            165

attgtgtctg ttttaatccc tatcttgtga tttaataatg taacgtaata tgcatgtaat      650

aagtaagccg agtgttgtgt tttcaataaa atagaatttg cttttgcaag ttctaatgct      710

tttcaaaaaa aaaaaaaaaa                                                  730

<210>  6
<211>  169
<212>  PRT
<213>  Allium chinense L.

<400>  6

Met Glu Gln Asn Ser Gly Thr Leu Ala Val Val Thr Asp Gly Ala Lys
1               5                   10                  15

Gly Ala Ala Lys Trp Arg Gly Lys Val His Ala Leu Leu Pro Asn Thr
            20                  25                  30

Lys Pro Glu Gln Ala Trp Thr Leu Leu Lys Asp Phe Ile Asn Leu His
        35                  40                  45

Lys Ile Met Pro Ser Leu Ser Val Cys Glu Leu Val Glu Gly Glu Ala
    50                  55                  60

Asn Val Val Gly Cys Val Arg His Val Lys Gly Ile Met His Pro Met
65                  70                  75                  80

Glu Glu Glu Phe Trp Ala Lys Glu Lys Leu Val Ala Leu Asp Asp Lys
                85                  90                  95

Asn Met Ser Cys Ser Tyr Ile Phe Val Glu Cys Phe Thr Gly Tyr Glu
            100                 105                 110

Asp Tyr Thr Ala Thr Met Gln Ile Val Glu Gly Ser Glu His Lys Gly
        115                 120                 125

Cys Arg Phe Asp Trp Ser Phe Gln Cys Lys Tyr Ile Glu Gly Met Thr
    130                 135                 140

Glu Ser Ala Phe Thr Asp Val Leu Gln His Trp Ala Thr Glu Ile Gly
145                 150                 155                 160

Gln Lys Ile Glu Glu Ile Cys Asn Ala
                165

<210> 7
<211> 739
<212> DNA

<213>  Allium cepa L.

<220>
<221>  CDS
<222>  (55)..(561)
<223>  P

<220>
<221>  polyA_site
<222>  (724)..(739)
<223>  P

<400>  7
acacaattca gactcacatt acgttatatc aagaagattg tccaatcaga aaaa atg        57
                                                            Met
                                                            1

gag cta aat cct ggt gca cct gct gta gtc gct gat agt gct aac gga       105
Glu Leu Asn Pro Gly Ala Pro Ala Val Val Ala Asp Ser Ala Asn Gly
            5                   10                  15

gct cga aaa tgg agc ggc aaa gtc cat gct ttg ctt cca aat aca aag       153
Ala Arg Lys Trp Ser Gly Lys Val His Ala Leu Leu Pro Asn Thr Lys
        20                  25                  30

cca gag caa gca tgg aca cta cta aaa gac ttt att aac ctt cac aag       201
Pro Glu Gln Ala Trp Thr Leu Leu Lys Asp Phe Ile Asn Leu His Lys
    35                  40                  45

gtc atg cct tcg ttg tca gtc tgt gaa ctg gta gaa ggt gag gcc aat       249
Val Met Pro Ser Leu Ser Val Cys Glu Leu Val Glu Gly Glu Ala Asn
50                  55                  60                  65

gtt gtt ggt tgt gtt cgc tac gtt aaa ggt ata atg cac cca ata gaa       297

Val Val Gly Cys Val Arg Tyr Val Lys Gly Ile Met His Pro Ile Glu
                70                  75                  80

gag gaa ttt tgg gcc aag gag aag ctg gtg gcg ctg gat aat aag aac      345
Glu Glu Phe Trp Ala Lys Glu Lys Leu Val Ala Leu Asp Asn Lys Asn
            85                  90                  95

atg agc tac agt tat att ttt act gag tgt ttt aca ggg tac gag gat      393
Met Ser Tyr Ser Tyr Ile Phe Thr Glu Cys Phe Thr Gly Tyr Glu Asp
        100                 105                 110

tac acg gct acc atg caa ata gtg gag ggt cct gag cac aag gga agt      441
Tyr Thr Ala Thr Met Gln Ile Val Glu Gly Pro Glu His Lys Gly Ser
    115                 120                 125

aga ttt gac tgg tct ttt cag tgc aag tat atc gag ggt atg act gaa      489
Arg Phe Asp Trp Ser Phe Gln Cys Lys Tyr Ile Glu Gly Met Thr Glu
130                 135                 140                 145

tct gca ttc acc gag att ctg cag cat tgg gct act gag ata ggt cag      537
Ser Ala Phe Thr Glu Ile Leu Gln His Trp Ala Thr Glu Ile Gly Gln
                150                 155                 160

aaa atc gaa gag gtt tgc agt gct tgatcatgaa tatcggtttt cagtgctgtg      591
Lys Ile Glu Glu Val Cys Ser Ala
            165

atgcattatg tgtcttttaa accttgtctt gtgatataat aaagtaacgt aatatgtgca      651

tgtaataagt aagactgagt gttgtgtgtt caataaaaaa gaatttgctt tttgcaagtt      711

ctagtgcttt tcaaaaaaaa aaaaaaaa                                        739

<210>  8
<211>  169

<212>  PRT
<213>  Allium cepa L.

<400>  8

Met Glu Leu Asn Pro Gly Ala Pro Ala Val Val Ala Asp Ser Ala Asn
1               5                   10                  15

Gly Ala Arg Lys Trp Ser Gly Lys Val His Ala Leu Leu Pro Asn Thr
            20                  25                  30

Lys Pro Glu Gln Ala Trp Thr Leu Leu Lys Asp Phe Ile Asn Leu His
        35                  40                  45

Lys Val Met Pro Ser Leu Ser Val Cys Glu Leu Val Glu Gly Glu Ala
    50                  55                  60

Asn Val Val Gly Cys Val Arg Tyr Val Lys Gly Ile Met His Pro Ile
65                  70                  75                  80

Glu Glu Glu Phe Trp Ala Lys Glu Lys Leu Val Ala Leu Asp Asn Lys
                85                  90                  95

Asn Met Ser Tyr Ser Tyr Ile Phe Thr Glu Cys Phe Thr Gly Tyr Glu
            100                 105                 110

Asp Tyr Thr Ala Thr Met Gln Ile Val Glu Gly Pro Glu His Lys Gly
        115                 120                 125

Ser Arg Phe Asp Trp Ser Phe Gln Cys Lys Tyr Ile Glu Gly Met Thr
    130                 135                 140

Glu Ser Ala Phe Thr Glu Ile Leu Gln His Trp Ala Thr Glu Ile Gly
145                 150                 155                 160

Gln Lys Ile Glu Glu Val Cys Ser Ala
                165

<210>  9
<211>  648
<212>  DNA
<213>  Allium ampeloprasum L.

<220>
<221>  CDS
<222>  (1)..(492)
<223>  P

<220>
<221>  polyA_site
<222>  (632)..(648)
<223>  P

<400>  9
atg gcg caa aat cct ggt gtg cct gct gta gcc act gag cca aaa tgg        48
Met Ala Gln Asn Pro Gly Val Pro Ala Val Ala Thr Glu Pro Lys Trp
1               5                   10                  15

aca ggc aag gtc agt gca tcg ctt cca aat aca aag gca gag caa gca        96

Thr Gly Lys Val Ser Ala Ser Leu Pro Asn Thr Lys Ala Glu Gln Ala
            20                  25                  30

tgg aca cta cta aaa gac ttt gtt aac ctt gac aag gtt atg cct tcg 144
Trp Thr Leu Leu Lys Asp Phe Val Asn Leu Asp Lys Val Met Pro Ser
        35                  40                  45

ttg tcg gtt tgt gaa ctg gta gaa ggt aaa ccc aat gct gtt ggt tgt 192
Leu Ser Val Cys Glu Leu Val Glu Gly Lys Pro Asn Ala Val Gly Cys
    50                  55                  60

act cgc tac gtt aaa ggt atg atg cac cca atg gaa gtg gaa ttt tgg 240
Thr Arg Tyr Val Lys Gly Met Met His Pro Met Glu Val Glu Phe Trp
65                  70                  75                  80

gcc aac gag cag ctg gtg gag ctg gat gac gag acc atg acc tac agt 288
Ala Asn Glu Gln Leu Val Glu Leu Asp Asp Glu Thr Met Thr Tyr Ser
                85                  90                  95

tat att ttt act aag gcc ttt aca ggg tat gag ggt tac atg ggt acc 336
Tyr Ile Phe Thr Lys Ala Phe Thr Gly Tyr Glu Gly Tyr Met Gly Thr
            100                 105                 110

atg caa ctt gtg gag gaa agc gat cag aag gga act aga ttt gac tgg 384
Met Gln Leu Val Glu Glu Ser Asp Gln Lys Gly Thr Arg Phe Asp Trp
        115                 120                 125

tct ttt cag tgc aag tac att gag ggt gtg act gcc act tca ttc gct 432
Ser Phe Gln Cys Lys Tyr Ile Glu Gly Val Thr Ala Thr Ser Phe Ala
    130                 135                 140

gct gtt ctg cag att tgg gca gat gag att gcc cag aaa att gaa gag 480
Ala Val Leu Gln Ile Trp Ala Asp Glu Ile Ala Gln Lys Ile Glu Glu
145                 150                 155                 160

att tgc aaa gca tgatcatgaa tatgggtcaa tttgtgatgc tgtgtgcatg 532

Ile Cys Lys Ala

tgtgttttcc ttctgtcttg tgatgtaatg aaagtaacgt aattccagat gcatgtaatc     592

tgtagtgctt gtgttttcaa taaataagaa tttgctttca aaaaaaaaaa aaaaaa         648

<210>  10
<211>  164
<212>  PRT
<213>  Allium ampeloprasum L.

<400>  10

Met Ala Gln Asn Pro Gly Val Pro Ala Val Ala Thr Glu Pro Lys Trp
1               5                   10                  15

Thr Gly Lys Val Ser Ala Ser Leu Pro Asn Thr Lys Ala Glu Gln Ala
            20                  25                  30

Trp Thr Leu Leu Lys Asp Phe Val Asn Leu Asp Lys Val Met Pro Ser
        35                  40                  45

Leu Ser Val Cys Glu Leu Val Glu Gly Lys Pro Asn Ala Val Gly Cys
    50                  55                  60

Thr Arg Tyr Val Lys Gly Met Met His Pro Met Glu Val Glu Phe Trp
65                  70                  75                  80

Ala Asn Glu Gln Leu Val Glu Leu Asp Asp Glu Thr Met Thr Tyr Ser

                85                  90                  95

Tyr Ile Phe Thr Lys Ala Phe Thr Gly Tyr Glu Gly Tyr Met Gly Thr
            100                 105                 110

Met Gln Leu Val Glu Glu Ser Asp Gln Lys Gly Thr Arg Phe Asp Trp
        115                 120                 125

Ser Phe Gln Cys Lys Tyr Ile Glu Gly Val Thr Ala Thr Ser Phe Ala
    130                 135                 140

Ala Val Leu Gln Ile Trp Ala Asp Glu Ile Ala Gln Lys Ile Glu Glu
145                 150                 155                 160

Ile Cys Lys Ala

<210> 11
<211> 737
<212> DNA
<213> Allium cepa L.

<220>
<221> CDS
<222> (53)..(559)
<223> P

<220>
<221> polyA_site

<222>  (722)..(737)
<223>  P

<400>  11
acaattcaga ctcacattac gttatatcaa gaagattgtc caatcagaaa aa atg gag      58
                                                          Met Glu
                                                          1

cta aat cct ggt gca cct gct gta gtc gct gat agt gct aac gga gct      106
Leu Asn Pro Gly Ala Pro Ala Val Val Ala Asp Ser Ala Asn Gly Ala
        5                   10                  15

cga aaa tgg agc ggc aaa gtc cat gct ttg ctt cca aat aca aag cca      154
Arg Lys Trp Ser Gly Lys Val His Ala Leu Leu Pro Asn Thr Lys Pro
    20                  25                  30

gag caa gca tgg aca cta cta aaa gac ttt att aac ctt cac aag gtc      202
Glu Gln Ala Trp Thr Leu Leu Lys Asp Phe Ile Asn Leu His Lys Val
35                  40                  45                  50

atg cct tcg ttg tca gtc tgt gaa ctg gta gaa ggt gag gcc aat gtt      250
Met Pro Ser Leu Ser Val Cys Glu Leu Val Glu Gly Glu Ala Asn Val
                55                  60                  65

gtt ggt tgt gtt cgc tac gtt aaa ggt ata atg cac cca ata gaa gag      298
Val Gly Cys Val Arg Tyr Val Lys Gly Ile Met His Pro Ile Glu Glu
            70                  75                  80

gaa ttt tgg gcc aag gag aag ctg gtg gcg ctg gat aat aag aac atg      346
Glu Phe Trp Ala Lys Glu Lys Leu Val Ala Leu Asp Asn Lys Asn Met
        85                  90                  95

agc tac agt tat att ttt act gag tgt ttt aca ggg tac gag gat tac      394
Ser Tyr Ser Tyr Ile Phe Thr Glu Cys Phe Thr Gly Tyr Glu Asp Tyr
    100                 105                 110

acg gct acc atg caa ata gtg gag ggt cct gag cac aag gga agt aga 442
Thr Ala Thr Met Gln Ile Val Glu Gly Pro Glu His Lys Gly Ser Arg
115                 120                 125                 130

ttt gac tgg tct ttt cag tgc aag tat atc gag ggt atg act gaa tct 490
Phe Asp Trp Ser Phe Gln Cys Lys Tyr Ile Glu Gly Met Thr Glu Ser
                135                 140                 145

gca ttc acc gag att ctg cag cat tgg gct act gag ata ggt cag aaa 538
Ala Phe Thr Glu Ile Leu Gln His Trp Ala Thr Glu Ile Gly Gln Lys
            150                 155                 160

atc gaa gag gtt tgc agt gct tgatcatgaa tatcggtttt cagtgctgtg 589
Ile Glu Glu Val Cys Ser Ala
        165

atgcattatg tgtcttttaa accttgtctt gtgatataat aaagtaacgt aatatgtgca 649

tgtaataagt aagactgagt gttgtgtgtt caataaaaaa gaatttgctt tttgcaagtt 709

ctagtgcttt tcaaaaaaaa aaaaaaaa 737

<210> 12
<211> 169
<212> PRT
<213> Allium cepa L.

<400> 12

Met Glu Leu Asn Pro Gly Ala Pro Ala Val Val Ala Asp Ser Ala Asn
1               5                   10                  15

Gly Ala Arg Lys Trp Ser Gly Lys Val His Ala Leu Leu Pro Asn Thr

            20                  25                  30

Lys Pro Glu Gln Ala Trp Thr Leu Leu Lys Asp Phe Ile Asn Leu His
        35                  40                  45

Lys Val Met Pro Ser Leu Ser Val Cys Glu Leu Val Glu Gly Glu Ala
    50                  55                  60

Asn Val Val Gly Cys Val Arg Tyr Val Lys Gly Ile Met His Pro Ile
65                  70                  75                  80

Glu Glu Glu Phe Trp Ala Lys Glu Lys Leu Val Ala Leu Asp Asn Lys
                85                  90                  95

Asn Met Ser Tyr Ser Tyr Ile Phe Thr Glu Cys Phe Thr Gly Tyr Glu
            100                 105                 110

Asp Tyr Thr Ala Thr Met Gln Ile Val Glu Gly Pro Glu His Lys Gly
        115                 120                 125

Ser Arg Phe Asp Trp Ser Phe Gln Cys Lys Tyr Ile Glu Gly Met Thr
    130                 135                 140

Glu Ser Ala Phe Thr Glu Ile Leu Gln His Trp Ala Thr Glu Ile Gly
145                 150                 155                 160

Gln Lys Ile Glu Glu Val Cys Ser Ala

                165

<210>  13
<211>  661
<212>  DNA
<213>  Allium ampeloprasum L.

<220>
<221>  CDS
<222>  (1)..(492)
<223>  P

<220>
<221>  polyA_site
<222>  (637)..(661)
<223>  P

<400>  13
atg gcg caa aat cct ggt gtg cct gct gtt gcc act gag cca aaa tgg      48
Met Ala Gln Asn Pro Gly Val Pro Ala Val Ala Thr Glu Pro Lys Trp
1               5                   10                  15

aca ggc aag gtc agt gca tcg ctt cca aat aca aag cca gag caa gca      96
Thr Gly Lys Val Ser Ala Ser Leu Pro Asn Thr Lys Pro Glu Gln Ala
            20                  25                  30

tgg aca ctg cta aaa gac ttt gtt aac ctt gac aag gtt atg cct tca      144
Trp Thr Leu Leu Lys Asp Phe Val Asn Leu Asp Lys Val Met Pro Ser
        35                  40                  45

ttg tca gtt tgt gaa ctt gta gaa ggt gaa ccc aat gcc gtt ggt tgt      192
Leu Ser Val Cys Glu Leu Val Glu Gly Glu Pro Asn Ala Val Gly Cys
    50                  55                  60

act cgc tac gtt aaa ggt atg atg cac cca atg gaa gtg gaa ttt tgg 240
Thr Arg Tyr Val Lys Gly Met Met His Pro Met Glu Val Glu Phe Trp
65                  70                  75                  80

gcc aac gag cag ctg gtg gag ctg gat gac gag acc atg acc tac agt 288
Ala Asn Glu Gln Leu Val Glu Leu Asp Asp Glu Thr Met Thr Tyr Ser
                85                  90                  95

tat att ttt act aag gcc ttt aca ggg tat gag ggt tac atg ggt acc 336
Tyr Ile Phe Thr Lys Ala Phe Thr Gly Tyr Glu Gly Tyr Met Gly Thr
            100                 105                 110

atg caa ctt gtg gag gaa agc gat cag aag gga act agg ttt gac tgg 384
Met Gln Leu Val Glu Glu Ser Asp Gln Lys Gly Thr Arg Phe Asp Trp
        115                 120                 125

tct ttt cag tgc aag tac att gag ggt gtg act gcc aca tca ttc gct 432
Ser Phe Gln Cys Lys Tyr Ile Glu Gly Val Thr Ala Thr Ser Phe Ala
    130                 135                 140

gct gtt ctg cag att tgg gca gat gag att gcc cag aaa att gaa gag 480
Ala Val Leu Gln Ile Trp Ala Asp Glu Ile Ala Gln Lys Ile Glu Glu
145                 150                 155                 160

att tgc aaa gca tgatcatgaa tatgggtcaa tttgtgatgc tgtgtgcatg 532
Ile Cys Lys Ala

tgtgttttcc attctgtctt gtgatgtaat gaagtaacgt aattaccaga tgcatgtaat 592

ctgtagtggc tgtgttttca ataaataaga atttgctttc ttgcaaaaaa aaaaaaaaaa 652

aaaaaaaaa 661

<210>  14
<211>  164
<212>  PRT
<213>  Allium ampeloprasum L.

<400>  14

Met Ala Gln Asn Pro Gly Val Pro Ala Val Ala Thr Glu Pro Lys Trp
1               5                   10                  15

Thr Gly Lys Val Ser Ala Ser Leu Pro Asn Thr Lys Pro Glu Gln Ala
            20                  25                  30

Trp Thr Leu Leu Lys Asp Phe Val Asn Leu Asp Lys Val Met Pro Ser
        35                  40                  45

Leu Ser Val Cys Glu Leu Val Glu Gly Glu Pro Asn Ala Val Gly Cys
    50                  55                  60

Thr Arg Tyr Val Lys Gly Met Met His Pro Met Glu Val Glu Phe Trp
65                  70                  75                  80

Ala Asn Glu Gln Leu Val Glu Leu Asp Asp Glu Thr Met Thr Tyr Ser
                85                  90                  95

Tyr Ile Phe Thr Lys Ala Phe Thr Gly Tyr Glu Gly Tyr Met Gly Thr
            100                 105                 110

Met Gln Leu Val Glu Glu Ser Asp Gln Lys Gly Thr Arg Phe Asp Trp

115                 120                 125

Ser Phe Gln Cys Lys Tyr Ile Glu Gly Val Thr Ala Thr Ser Phe Ala
    130                 135                 140

Ala Val Leu Gln Ile Trp Ala Asp Glu Ile Ala Gln Lys Ile Glu Glu
145                 150                 155                 160

Ile Cys Lys Ala

<210>  15
<211>  726
<212>  DNA
<213>  Allium ampeloprasum L.

<220>
<221>  CDS
<222>  (57)..(563)
<223>  P

<220>
<221>  polyA_site
<222>  (717)..(726)
<223>  P

<400>  15
acacacaact cagacccaca tttcgttgta tttagtagat tattcagtca ggaaaa atg      59
                                                              Met
                                                              1

atg aca tat cct gga aat cgt gct gta gcc act gat ggt gcc aaa gaa 107
Met Thr Tyr Pro Gly Asn Arg Ala Val Ala Thr Asp Gly Ala Lys Glu
            5                   10                  15

gct cca aaa tgg aaa ggc aaa gcc tat gcc ttg ctt cca aat aca aag 155
Ala Pro Lys Trp Lys Gly Lys Ala Tyr Ala Leu Leu Pro Asn Thr Lys
        20                  25                  30

cca gag cac gcg tgg aaa cta cta aaa gac ttc att aac ctt cac aag 203
Pro Glu His Ala Trp Lys Leu Leu Lys Asp Phe Ile Asn Leu His Lys
    35                  40                  45

acc atg cca tcg ctg tca gtt tgt gaa ctg gta gaa ggt gag gtc aat 251
Thr Met Pro Ser Leu Ser Val Cys Glu Leu Val Glu Gly Glu Val Asn
50                  55                  60                  65

gct gta ggt tgt gtt cgt cat gtt aaa ggt ata atg cat cca atg gag 299
Ala Val Gly Cys Val Arg His Val Lys Gly Ile Met His Pro Met Glu
                70                  75                  80

cag gag ttt tgg gct aag gag aag ctg gtg gca gtc gat gac aag gcc 347
Gln Glu Phe Trp Ala Lys Glu Lys Leu Val Ala Val Asp Asp Lys Ala
            85                  90                  95

atg agc tac agt tat att ttt act gag tgt ttt aca ggg tac gag gat 395
Met Ser Tyr Ser Tyr Ile Phe Thr Glu Cys Phe Thr Gly Tyr Glu Asp
        100                 105                 110

tac acg gcc acc atg caa att atg gat gga tgc gag cat aag gga agc 443
Tyr Thr Ala Thr Met Gln Ile Met Asp Gly Cys Glu His Lys Gly Ser
    115                 120                 125

aga ttt gag tgg tcc ttc cag tgt aac tac atc gag ggt atg act gaa 491
Arg Phe Glu Trp Ser Phe Gln Cys Asn Tyr Ile Glu Gly Met Thr Glu
130                 135                 140                 145

tct gcc ttc act gac att ctg cag cat tgg acc act gag att ggt cag 539
Ser Ala Phe Thr Asp Ile Leu Gln His Trp Thr Thr Glu Ile Gly Gln
                150                 155                 160

aaa att gaa gag att tgc agt gct tgattatgaa tatcggttta tgctgtgatg 593
Lys Ile Glu Glu Ile Cys Ser Ala
            165

caatgtgtgt gtattaatcc ctgccttgtg atgtgataaa ataacttaat atgtcatatg 653

catgtaatag gcaagccagg gtggtgttgt gttctcaata aaaagcattt gctttttgca 713

taaaaaaaaa aaa 726

<210> 16
<211> 169
<212> PRT
<213> Allium ampeloprasum L.

<400> 16

Met Met Thr Tyr Pro Gly Asn Arg Ala Val Ala Thr Asp Gly Ala Lys
1               5                   10                  15

Glu Ala Pro Lys Trp Lys Gly Lys Ala Tyr Ala Leu Leu Pro Asn Thr
            20                  25                  30

Lys Pro Glu His Ala Trp Lys Leu Leu Lys Asp Phe Ile Asn Leu His
        35                  40                  45

Lys Thr Met Pro Ser Leu Ser Val Cys Glu Leu Val Glu Gly Glu Val

50                  55                  60

Asn Ala Val Gly Cys Val Arg His Val Lys Gly Ile Met His Pro Met
65                  70                  75                  80

Glu Gln Glu Phe Trp Ala Lys Glu Lys Leu Val Ala Val Asp Asp Lys
                85                  90                  95

Ala Met Ser Tyr Ser Tyr Ile Phe Thr Glu Cys Phe Thr Gly Tyr Glu
            100                 105                 110

Asp Tyr Thr Ala Thr Met Gln Ile Met Asp Gly Cys Glu His Lys Gly
        115                 120                 125

Ser Arg Phe Glu Trp Ser Phe Gln Cys Asn Tyr Ile Glu Gly Met Thr
    130                 135                 140

Glu Ser Ala Phe Thr Asp Ile Leu Gln His Trp Thr Thr Glu Ile Gly
145                 150                 155                 160

Gln Lys Ile Glu Glu Ile Cys Ser Ala
                165

<210>  17
<211>  20
<212>  DNA
<213>  Artificial

<220>
<223>  PCR primer

<400>  17
tggagggtcc tgagcacaag                                                    20

<210>  18
<211>  21
<212>  DNA
<213>  Artificial

<220>
<223>  PCR primer

<400>  18
tggaagaatt cgcggccgca g                                                  21

<210>  19
<211>  36
<212>  DNA
<213>  Artificial

<220>
<223>  PCR primer

<220>
<221>  modified_base
<222>  (24)..(24)
<223>  i

<220>
<221>  modified_base
<222>  (25)..(25)

<223>  i

<220>
<221>  modified_base
<222>  (29)..(29)
<223>  i

<220>
<221>  modified_base
<222>  (30)..(30)
<223>  i

<220>
<221>  modified_base
<222>  (34)..(34)
<223>  i

<220>
<221>  modified_base
<222>  (35)..(35)
<223>  i

<400>  19
ggccacgcgt cgactagtac gggnngggnn gggnng                                36

<210>  20
<211>  30
<212>  DNA
<213>  Artificial

<220>
<223>  PCR primer

<400>  20
ctcttcgatt ttctgaccta tctcagtagc                                        30

<210>  21
<211>  22
<212>  DNA
<213>  Artificial

<220>
<223>  PCR primer

<400>  21
atggagctaa atcctggtgc gc                                                22

<210>  22
<211>  22
<212>  DNA
<213>  Artificial

<220>
<223>  PCR primer

<400>  22
atggagcaaa attctggtac gc                                                22

<210>  23
<211>  15
<212>  DNA
<213>  Artificial

<220>
<223> PCR primer

<220>
<221> modified_base
<222> (3)..(3)
<223> i

<220>
<221> modified_base
<222> (6)..(6)
<223> i

<220>
<221> modified_base
<222> (9)..(9)
<223> i

<400> 23
ggngcnmgna artgg 15

<210> 24
<211> 22
<212> DNA
<213> Artificial

<220>
<223> PCR primer

<400> 24
atggagctaa atcctggtgc ac 22

<210>  25
<211>  25
<212>  DNA
<213>  Artificial

<220>
<223>  PCR primer

<400>  25
gaattttggg ccaaggagaa gctgg                                          25


<210>  26
<211>  25
<212>  DNA
<213>  Artificial

<220>
<223>  PCR primer

<400>  26
tcctcgtacc ctgtaaaaca ctcag                                          25


<210>  27
<211>  23
<212>  DNA
<213>  Artificial

<220>
<223>  PCR primer

<400>  27
atgatgacat atcctggaaa tcg                                            23

<210>  28
<211>  21
<212>  DNA
<213>  Artificial

<220>
<223>  PCR primer

<400>  28
cacacagcat cacaaattga c                                                21

<210>  29
<211>  22
<212>  DNA
<213>  Artificial

<220>
<223>  PCR primer

<400>  29
atggcgcaaa atcctggtgt gc                                               22

<210>  30
<211>  45
<212>  DNA
<213>  Artificial

<220>
<223>  PCR primer

<400>  30
aactggaaga attcgcggcc gcaggaattt tttttttttt ttttt                      45

## Description

### Technical Field:

The present invention relates to protein or polypeptide capable of converting 1-propenylsulfenic acid concerning the formation of lachrymatory factors, generated when plants such as onions are broken into pieces or cut, into lachrymatory factors, and also to DNA (lachrymatory factor-producing enzyme genes) encoding the protein or polypeptide.

The genes of the lachrymatory factor-producing enzyme of the present invention are usable for the repression of the formation of the lachrymatory factor, usable as a selection indicator for the materials, mates and the like in the development of a plant which generates only a reduced amount of lachrymatory factors generated by breaking or cutting the plant, and capable of providing the information for repressing the expression level of the enzyme, producing a large amount of the enzyme, producing a large amount of the lachrymatory factor and the like.

Concretely, the term "lachrymatory factor" (hereinafter referred to as "LF") in this specification indicates thiopropanal-S-oxide. The expression "to have the lachrymatory factor-producing enzymatic activity" indicates to have an effect of converting trans-1-propenylsulfenic acid, which is an estimate substrate of the lachrymatory factor-producing enzyme, into a lachrymatory factor or an effect of generating the lachrymatory factor from trans-S-1-propenyl-cystein sulfoxide (PeCSO) contained in onions or the like in the presence of an enzyme named alliinase.

### Background art:

The results of various investigations, on the lachrymatory factor formed when onions are broken into pieces or cut, were reported up to now. As for the generation and decomposition of the lachrymatory factor (LF) contained in onions or the like, it has been considered that when S-1-propenyl-cystein sulfoxide (PeCSO) is decomposed by alliinase, the lachrymatory factor is formed. Namely, it has been considered that when alliinase enzyme acts on PeCSO of the precursor, a stable lachrymatory factor is non-enzymatically formed through 1-propenylsulfenic acid.

However, according to the inventors' investigations, it has been found that when PeCSO is only decomposed with alliinase, the lachrymatory factor is not formed and, therefore, the participation of another enzyme (lachrymatory factor-producing enzyme) is indispensable. After intensive investigations mane under these circumstances, the inventors found that there is a new enzyme (lachrymatory factor-producing enzyme) which supposedly isomerizes the above-described sulfenic acid to form the lachrymatory factor. On the basis of this finding, the inventors found that the precursor is converted into a lachrymatory factor or into another flavor factor depending on the function of the enzyme. In addition, the inventors succeeded in developing a method for producing the lachrymatory factor-producing enzyme (lachrymatory substance-producing enzyme) and also in elucidating the physicochemical properties of the lachrymatory factor-producing enzyme. The inventors applied these finding for patent [Japanese Patent Unexamined Published Application (hereinafter referred to as "J. P. KOKAI") No. Hei 10-295373].

After intensive investigations made for the purpose of elucidating the structure of the lachrymatory factor-producing enzyme having the effect of forming the lachrymatory factor from PeCSO contained in onions or the like in the presence of alliinase, the inventors succeeded in elucidating isozymes of the lachrymatory factor-producing enzyme, the amino acid sequences thereof and the sequence of genes encoding onions. The inventors applied these findings for patent (see the pamphlet of International Publication No. 02/20808).

The above-described lachrymatory factor-producing enzyme is also generally contained in vegetables belonging to the genus Allium. The information of DNA encoding them is also useful for the genetic recombination and also the induction of variation and mating in the development of varieties of the vegetables. This information is thus useful for the production of vegetables which difficultly generate the lachrymatory factor even by breaking into pieces or cutting. In addition, this information is useful for the production of vegetables containing an increased amount of thiosulfinates (physiologically active substance) and reaction products thereof.

Further, by utilizing the information of DNA encoding the protein having the lachrymatory factor-producing enzymatic activity, it becomes possible to produce the enzyme in a large quantity by the technique of the genetic recombination or the like. This information is useful for the development of a technique of efficiently producing lachrymatory factors useful for the treatment of dry eyes or the like.

### Summary of the Invention:

The object of the present invention is to provide a gene of a lachrymatory factor-producing enzyme, in particular, a gene of a lachrymatory factor-producing enzyme contained in allium vegetables.

Another object of the present invention is to provide protein or polypeptides having a lachrymatory factor-producing enzymatic activity, in particular, protein or polypeptides having a lachrymatory factor-producing enzymatic activity contained in allium vegetables.

Still another object of the present invention is to provide a method for realizing the efficient production of isozymes of the lachrymatory factor-producing enzyme by a genetic recombination technique.

A further object of the present invention is to provide a means of realizing the repression of the expression of enzyme genes forming lachrymatory factors from a precursor thereof.

For attaining the above-described objects, the present invention is composed of the following technical means:
(1) DNA encoding protein or a polypeptide capable of converting 1-propenylsulfenic acid into a lachrymatory factor.
(2) The above-described DNA consisting of a base sequence of SEQ ID NO. 1, 5, 7, 9, 13 or 15, or any of the following (a) to (c):
   (a) DNA consisting of a base sequence of SEQ ID NO. 1, 5, 7, 9, 13 or 15 in which one or more of the bases are added, deleted or replaced,
   (b) DNA capable of being hybridized with DNA consisting of a base sequence of SEQ ID NO. 1, 5, 7, 9, 13 or 15 under stringent conditions, and
   (c) DNA consisting of a base sequence having a homology of at least 60 % with a base sequence of SEQ ID NO. 1, 5, 7, 9, 13 or 15.
(3) A primer usable for isolating gene of a lachrymatory factor-producing enzyme of SEQ ID NO. 25 of a vegetable of the genus allium and a method for isolating the gene with the primer.
(4) A recombinant vector containing the above-described DNA.
(5) A transformant containing a recombinant vector containing the above-described DNA.
(6) A method for preparing protein or a polypeptide having lachrymatory factor-producing enzymatic activity by culturing host cells transformed with a recombinant vector containing the above-described DNA and then separating protein or the polypeptide having lachrymatory factor-producing enzymatic activity produced in the medium or cells.
(7) Protein or a polypeptide having lachrymatory factor-producing enzymatic activity, which contains any of the following (a) to (c):
   (a) an amino acid sequence represented by SEQ ID NO. 2, 6, 8, 10, 14 or 16,
   (b) an amino acid sequence represented by SEQ ID NO. 2, 6, 8, 10, 14 or 16 in which one or more of the amino acids are added, deleted or replaced, and
   (c) an amino acid sequence having a homology of at least 65 % with an amino acid sequence of SEQ ID NO. 2, 6, 8, 10, 14 or 16.
(8) A nucleic acid molecule having a function of repressing the translation of mRNA on protein or polypeptide having lachrymatory factor-producing enzymatic activity.

The present invention includes a case wherein DNA in the above-described technical means excludes DNA consisting of a base sequence of SEQ ID NO. 11.

The present invention also includes a case wherein DNA in the above-described technical means excludes DNA encoding protein or a polypeptide containing an amino acid sequence of SEQ ID NO. 12.

The present invention also includes a case wherein protein or polypeptide in the above-described technical means excludes protein or polypeptide containing an amino acid sequence of SEQ ID NO. 12.

### Disclosure of the Invention:

In the present invention, the whole RNA was extracted from an allium vegetable, cDNA was synthesized with mRNA contained in the whole RNA as the template by RT-PCR method, a primer was designed on the basis of a base sequence of DNA (SEQ ID NO. 11) encoding the protein or polypeptide of the lachrymatory factor-producing enzyme of onion already obtained as described in a pamphlet of International Publication No. 02/20808, and the gene of the lachrymatory factor-producing enzyme was selectively synthesized with the cDNA as the template by PCR method. On the other hand, for allium vegetables which cannot selectively synthesize the gene of the lachrymatory factor-producing enzyme with the primer designed on the basis of the base sequence from onions, another primer effective for the isolation thereof was designed and the gene was selectively synthesized by the same method as that described above.

The lachrymatory factor-producing enzyme capable of producing the lachrymatory factor (thiopropanal S-oxide) from decomposition products obtained with PeCSO alliinase is important for improving the flavor and processability of vegetables. Thus, the determination of the gene of the lachrymatory factor-producing enzyme is extremely beneficial in the fields of producing the lachrymatory factor-producing enzyme and developing new varieties of plants.

Concretely, the methods for obtaining the sequences of the gene of the lachrymatory factor-producing enzyme are, for example, as follows:

### (1) (Extraction of all RNA · synthesis of cDNA)

cDNA is synthesized from all RNA in each allium vegetable. Any method known by those skilled in the art can be employed for this purpose. For instance, in an Example given below, a method wherein all RNA is extracted with RN easy Plant Mini Kit (QIAGEN Cat. No. 74903) and cDNA is synthesized with Ready-To-Go T-Primed First-Strand Kit (Amercham Biosciences code no. 27-9263-01) is employed.

### (2) (RACE method)

The gene of the lachrymatory factor-producing enzyme is sequenced by PCR-amplifying 3'-side region of the gene of the lachrymatory factor-producing enzyme of the allium vegetable with at least one primer containing the sequence in 3'-side region of the gene of the lachrymatory factor-producing enzyme of an onion and then PCR-amplifying 5'-side region of the gene of the lachrymatory factor-producing enzyme of the allium vegetable with at least one primer containing the sequence in 5'-side region of the gene of the lachrymatory factor-producing enzyme of an onion. The primers containing the sequence in 3'-side region of the gene of the lachrymatory factor-producing enzyme of the onion include an oligonucleotide of SEQ ID NO. 17, and the primers containing the sequence in 5'-side region of the gene of the lachrymatory factor-producing enzyme of the onion include an oligonucleotide of SEQ ID NO. 19. However, the primers are not limited to them.

### (3) (TA cloning method)

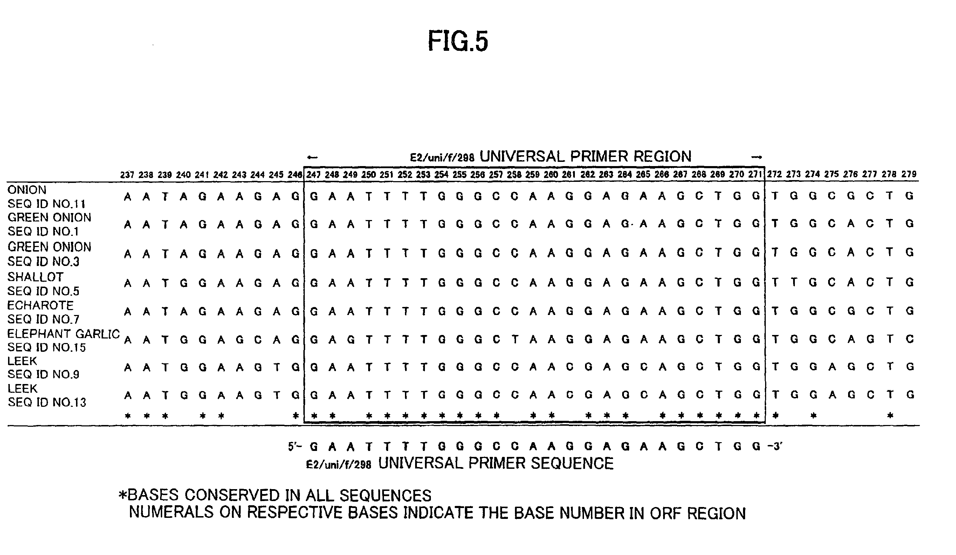

It was impossible to obtain RACE products of leek (A. ampeloprasum L.) only from the information of the genetic sequence of the lachrymatory factor-producing enzyme from onion. Therefore, regions in which the sequence of all the lachrymatory factor-producing enzyme genes was preserved in three kinds of vegetables, i. e. onion, shallot and green onion, were determined and then primer E2/uni/f/298 (SEQ ID NO. 25) designed on the basis of the sequence was used as the sense primer to make it possible to amplify the 3' side region of the cDNA. This primer is usable as a universal primer for isolating the gene of the lachrymatory factor-producing enzyme of a vegetable belonging to allium (the region of the universal primer is shown in Fig. 5).

The results that the amplification products could not be obtained by using the primer designed from the onion gene sequence were the same for 5' RACE. Thus, the amplification of 5' side region of the cDNA was made possible by designing primer E2/r/580-Le (SEQ ID NO. 28) from the base sequence of leek 3'RACE product obtained by using the above-described E2/uni/f/298 and using this primer as the antisense primer in 5'RACE-method.

It is a new finding that the cDNA full length sequence of the gene of the lachrymatory factor-producing enzyme gene of the leek could be determined by using the above-described primer E2/uni/f/298 in the 3' region.

The gene of the lachrymatory factor-producing enzyme of the present invention is a DNA encoding a protein or a polypeptide capable of converting 1-propenylsulfenic acid into the lachrymatory factor.

The proteins capable of catalyzing the reaction for converting PeCSO, which is a precursor for the lachrymatory factor, into the lachrymatory factor include alliinase and lachrymatory factor-producing enzymes. They are contained in allium vegetables which produce the lachrymatory factors by a physical damage such as cutting, e. g. onion, green onion, shallot, leek, echarote, elephant garlic and chive. The genes of the lachrymatory factor-producing enzyme include green onion DNA of SEQ ID NO. 1, shallot DNA of SEQ ID NO. 5, echarote DNA of SEQ ID NO. 7, leek DNA of SEQ ID NOS. 9 and 13, onion DNA of SEQ ID NO. 11 and elephant garlic DNA of SEQ ID NO. 15. However, the genes of the lachrymatory factor-producing enzymes are not limited to them. In the above-described base sequences of DNA, one or more bases may be added, deleted or replaced. For example, DNA may encode the proteins or polypeptides wherein in the amino acid sequences of SEQ ID NOS. 2, 6, 8, 10, 12, 14 and 16 corresponding to the above-described DNA, one or more amino acids may be added, deleted or replaced. Said proteins or polypeptides have a function of converting 1-propenylsulfenic acid into the lachrymatory factor.

It is known that in amino acid sequences, differences which exert no influence on the function thereof can be recognized among the strains or analogous ones. Among the sequences of them, coincidence of at least 90 %, or at least 95 %, of amino acids is recognized. This fact corresponds to that variation (addition, deletion or replacement) of not more than 10 or not more than 5 amino acids is recognized in 100 amino acids.

On the other hand, in the protein of the lachrymatory factor-producing enzyme of the present invention, the following fact was found from the results of the estimated amino acid sequence obtained by sequencing the gene of the lachrymatory factor-producing enzyme of several vegetables of the genus allium: the vegetables have the lachrymatory factor-producing enzymatic activity even when 35 amino acids in 100 amino acids are varied. This fact indicates that the variation of up to 35 % of the amino acid sequences is acceptable.

The genes of the lachrymatory factor-producing enzyme are DNA capable of hybridizing with DNA of a base sequence of the above-described SEQ ID NO. 1, 5, 7, 9, 13 or 15 under stringent conditions. The hybridizable DNA includes both DNA hybridizable with DNA of a base sequence of each SEQ ID number and complementary DNA. In other words, this DNA consists of a base sequence having a homology of at least 60 %, preferably at least 70 % and more preferably at least 75 % with the base sequence of the above-described SEQ ID No. 1, 5, 7, 9, 13 or 15.

### (Hybridization conditions of base sequence)

The expression "stringent conditions" in the present invention herein indicates such conditions that base sequence of SEQ ID NO. 1, 5, 7, 9, 13 or 15 or a part thereof is specifically hybridized with DNA and that nonspecific hybrid is not generated or detected. It is difficult to numerically express the stringent conditions. An example of the conditions is as follows: even when a hybrid is formed under such hybridization conditions that a hybridization buffer containing 30 % (v/v) deionized formaldehyde, 0.6 M of NaCl, 0.04 M of NaH₂PO₄, 2.5 mM of EDTA and 7 % of SDS is used at 42°C and then the formed hybrid is washed with 2 X SSC, 0.1 % SDS, the hybrid is still kept. The hybridization of nucleic acids can be performed according to, for example, Molecular Cloning: A laboratory manual (1989) Cold Spring Harbor Laboratory Press, New York, USA.

### (Homology in base sequence)

The homology in the base sequence is judged as follows: The alignment of the base sequence conducted prior to the judgment of the homology of bases in the sequences is conducted by using CLUSTAL W 1.81 DDBJ extended version (the algorism is conducted according to Gene 73, (1988) 237-244; CLUSTAL W by DDBJ) which is an internet analysis service of DNA Data Bank of Japan (http://www.ddbj.nig.ac.jp/E-mail/homology.html). The analysis parameter is kept default (gapdist: 8, maxdiv: 40, gapopen: 15, gapext: 6.66). The alignment results thus obtained are used for calculating the percentage of the number of the bases coincided in ORF based on the total number of the bases of ORF (gap region formed by the alignment is excluded) to calculate the homology of the bases in ORF.

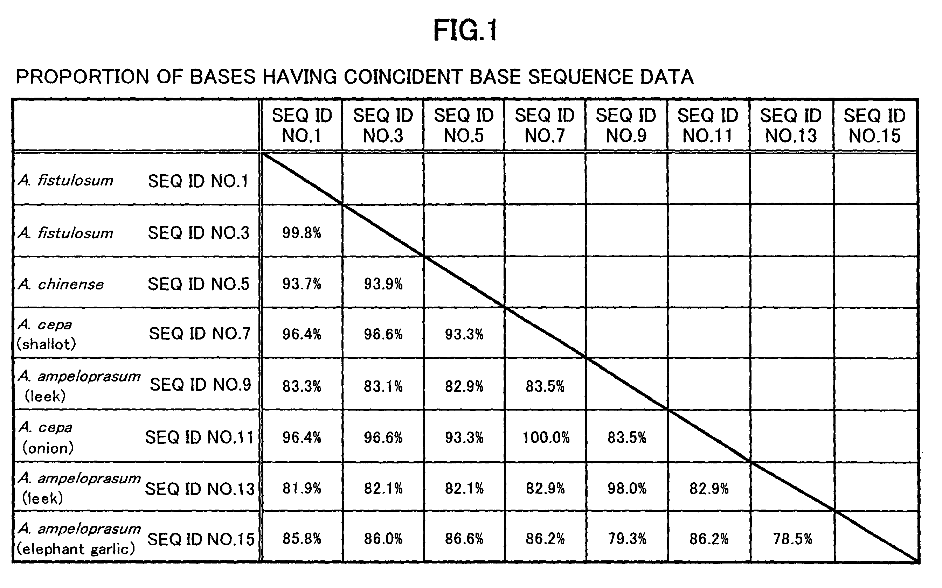

The homology in the base sequence of the base sequences of SEQ ID Nos. 1, 3, 5, 7, 9, 11, 13 and 15 is shown in Fig. 1. As a result, the homology in the base sequence between SEQ ID No. 15 (elephant garlic) and SEQ ID No. 13 (leek) was the lowest (78.5 %). Thus, the base sequences of the genes of the lachrymatory factor-producing enzymes have a high homology to each other and they form a family. DNA of SEQ ID NO. 3 is from green onion and has a homology of as high as 99.8 % to DNA of SEQ ID NO. 1. When the homology retrieval of the base sequence of a gene of the lachrymatory factor-producing enzyme of any of SEQ ID NOS. 1, 3, 5, 7, 9, 11, 13 and 15 was conducted according to BLAST search [the algorism is conducted according to J. Mol. Biol. Vol. 215, (1990), pp. 403-410. The analysis parameter is kept default (Expect: 10, Word size: 11, Low complexity filter: ON)] as compared with the gene sequence registered at Gen Bank, no gene sequence having a high homology was found except for the above-described genes of the lachrymatory factor-producing enzyme (only a homology of about 4 % was found to a part of the gene sequence data of Arabidopsis thaliana, yellow fruit fly and human being). From this fact, it is understood that the base sequence of the gene of the lachrymatory factor-producing enzyme is not homologous to any gene sequence reported until now and is utterly different from it.

The protein or polypeptide having the lachrymatory factor-producing enzymatic activity contains, for example, an amino acid sequence having at least 65 % homology to the amino acid sequence of SEQ ID NO. 2, 6, 8, 10, 14 or 16.

### (Homology of amino acid sequences)

The homology of amino acid sequences is judged as follows: the alignment of the amino acid sequence conducted prior to the judgment of the homology of amino acids in the sequences is conducted according to CLUSTAL W 1.81 DDBJ extended version (the algorism is conducted according to Gene 73, (1988) 237-244; CLUSTAL W by DDBJ) which is an internet analysis service of DNA Data Bank of Japan DDBJ (http://www.ddbj.nig.ac.jp/E-mail/homology.html). The analysis parameter is kept default (gapdist: 8, maxdiv: 40, gapopen: 10, gapext: 0.2). The alignment results thus obtained are used for calculating the percentage of the number of the coincided amino acids based on the total number of the amino acids (gap region formed by the alignment is excluded) to calculate the homology of the amino acids.

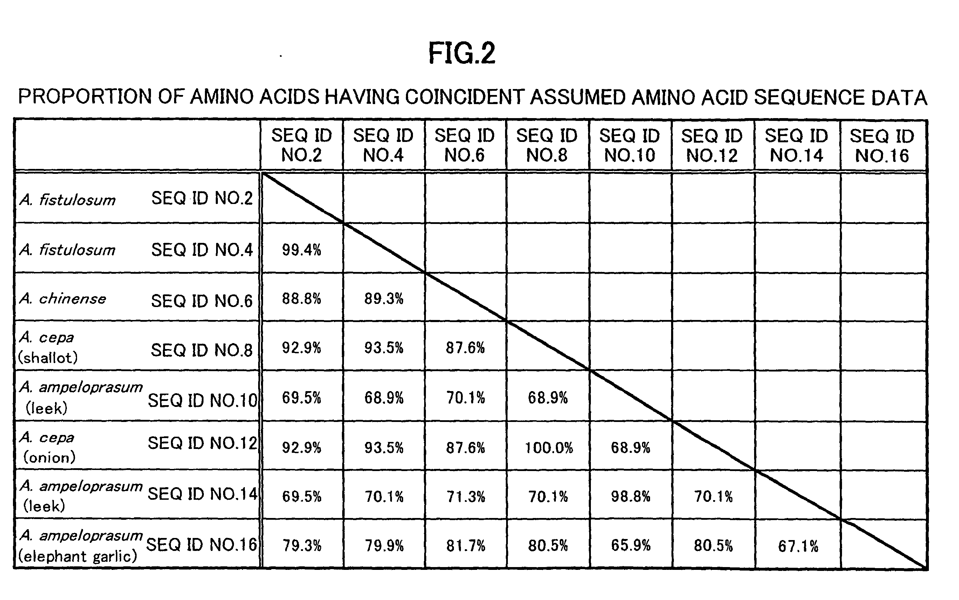

The homology of the estimated amino acid sequence in the estimated amino acid sequences of SEQ ID Nos. 2, 4, 6, 8, 10, 12, 14 and 16 is shown in Fig. 2. As a result, the homology in the estimated amino acid sequence between SEQ ID No. 16 (elephant garlic) and SEQ ID No. 10 (leek) was the lowest (65.9 %). Thus, the amino acid sequences of the protein of the lachrymatory factor-producing enzymes and the like have a high homology to each other and they form a family. Amino acid sequence of SEQ ID NO. 4 corresponds to that of green onion DNA of of SEQ ID NO. 3.

When the homology retrieval of the estimated amino acid sequence of a gene of the lachrymatory factor-producing enzyme of any of SEQ ID NOS. 2, 4, 6, 8, 10, 12, 14 and 16 was conducted according to BLAST search [the algorism is conducted according to J. Mol. Biol. Vol. 215, (1990), pp. 403-410. The analysis parameter is kept default (Expect: 10, Word size: 3, Low complexity filter: ON)] as compared with the amino acid sequence registered at Gen Bank to find that the homology of even the amino acid sequence having the highest homology (except for the estimated amino acid sequence of the genes of the lachrymatory factor-producing enzyme) was lower than 35 % (estimated amino acid sequences of genes, having unknown function, of Arabidopsis thaliana and rice plant). From this fact, it is understood that the estimated amino acid sequence of the gene of the lachrymatory factor-producing enzyme is not highly homologous to any amino acid sequence reported until now and is utterly different from it.

It is well known in the art that even when one or more amino acids are added, deleted or replaced in an amino acid sequence of protein or polypeptides having a specified function or physiological activity, said proteins or polypeptides keep the function and physiological activity. The present invention includes thus modified DNA fractions capable of encoding protein having the lachrymatory factor-producing enzymatic activity.

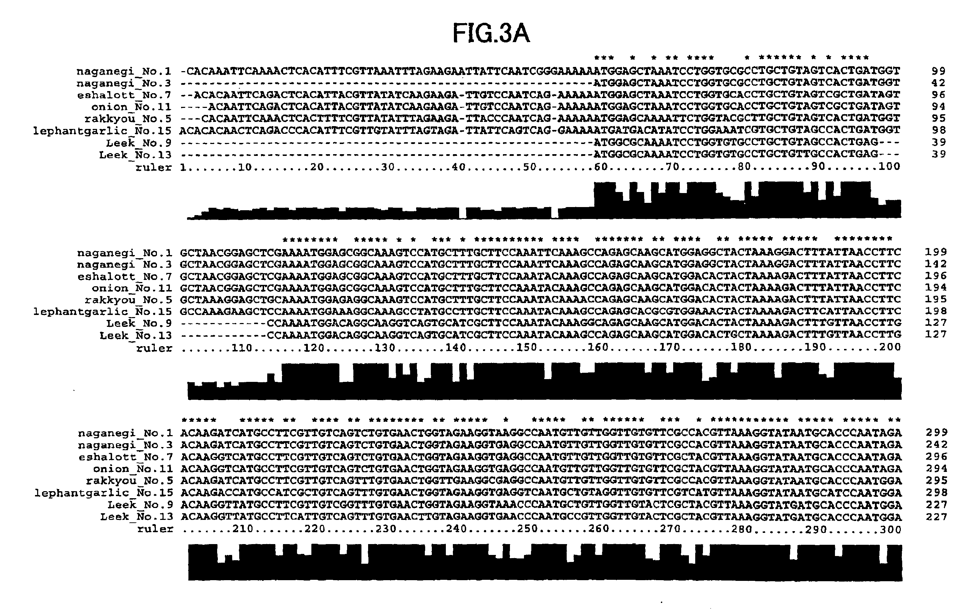

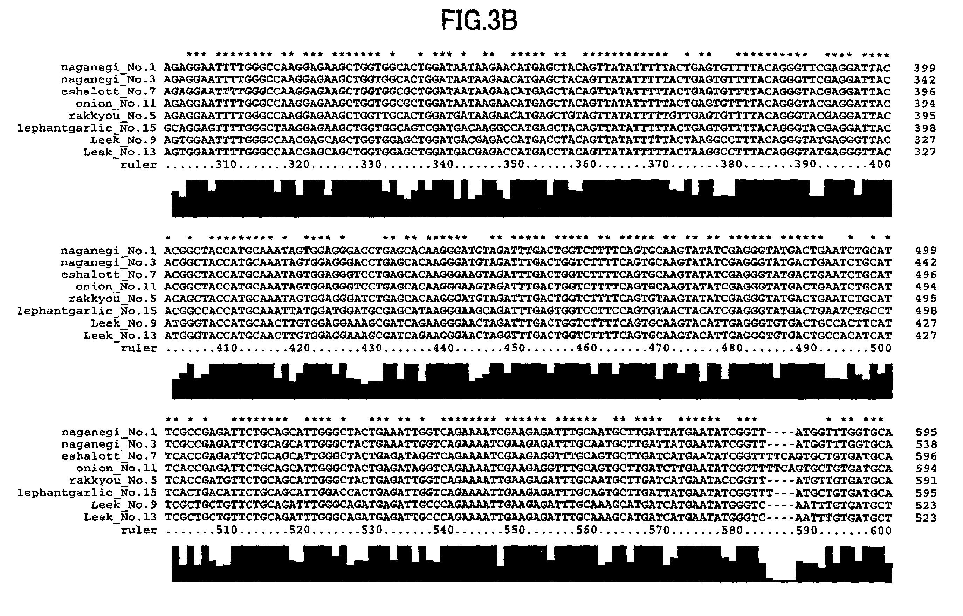

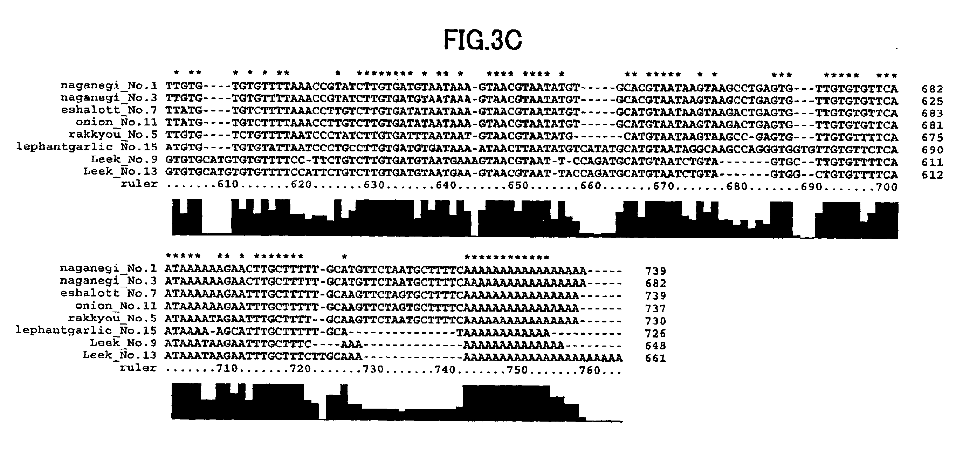

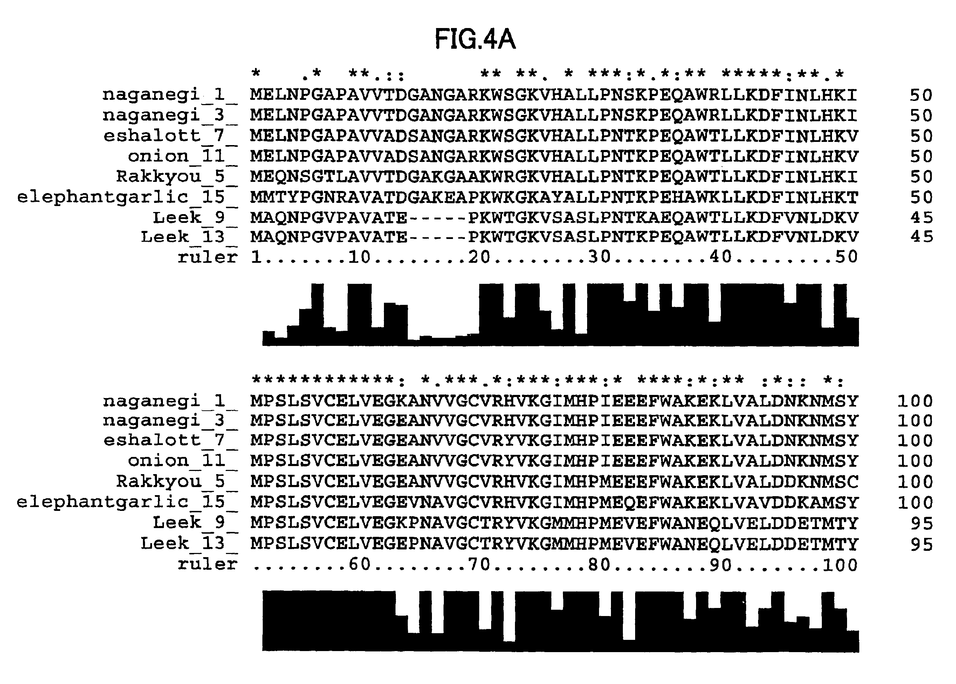

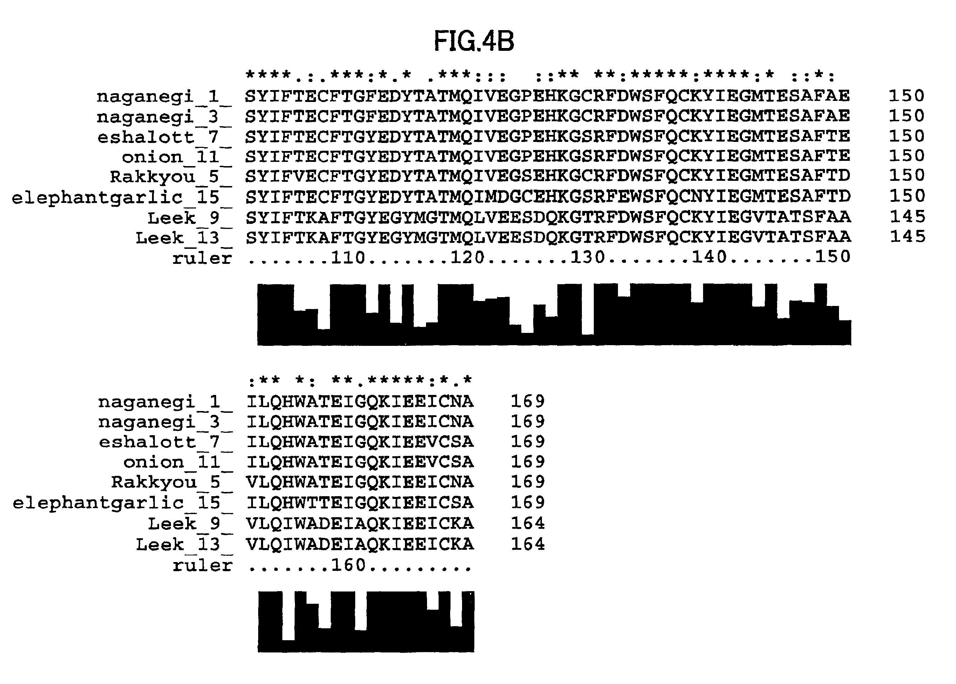

The alignment data of the base sequences of SEQ ID NOS. 1, 3, 5, 7, 9, 11, 13 and 15 are shown in Figs. 3A to 3C, and the alignment data of the amino acid sequences of SEQ ID NOS. 2, 4, 6, 8, 10, 12, 14 and 16 are shown in Figs. 4A and 4B. Figs. 3A to 3C show that the conservativeness of the bases tends to be low in a region of about 50 bases from 5' terminal of ORF. Particularly in leeks, 15 bases were deleted in this region. In onions, the presence of isozymes, in which the peptides derived from this region were removed after the translation into amino acids, is known. A region having a particularly low conservativeness was not found in cDNA of the gene of lachrymatory factor-producing enzyme in regions other than this region.

The genes of the lachrymatory factor-producing enzymes of the present invention are useful for, for example, a method for repressing the production of the lachrymatory factor, a method for selecting vegetables to be used for the crossbreeding by using them as indicators, a method for producing new varieties of vegetables having a reduced lachrymatory factor-producing enzymatic activity or vegetables having an increased amount of physiologically activating substances, and a method for producing a large amount of the lachrymatory factor-producing enzyme by the genetic recombination technique.

In the present invention, cDNA is synthesized from mRNA of the gene of the lachrymatory factor-producing enzyme by RT-PCR and the sequence of the sense strand thereof is determined. The sequence of the antisense strand is unconditionally determined from the sequence of the sense strand. Accordingly, DNA of the present invention contains the sequences of both sense strand and antisense strand. Namely, the present invention includes DNA having the antisense sequence to the base sequence of SEQ ID NOS. 1, 3, 5, 7, 9, 13 and 15 or fractions thereof.

The gene of the lachrymatory factor-producing enzyme of the present invention can be used for, for example, the following purposes:

### (1) Production of lachrymatory factor-producing enzyme:

The gene of the lachrymatory factor-producing enzyme of the present invention can be integrated into a suitable expression vector to obtain a recombinant vector.

The vector to be used is not particularly limited so far as it can be autonomously replicated in the host cells, it has an insertion site for integrating the above-described DNA, i. e. the gene of the lachrymatory factor-producing enzyme, and it also has a region in which the integrated DNA is expressed in the host cells. The expression vectors usable herein include, for example, pGEX-4T-3 (Amercham Bioscience Co.) having a multi-cloning site in the downstream of the sequence of glutathione S transferase (GST) gene.

A codon is converted in some cases so as to tally with the biological species, into which the codon is to be integrated, to accelerate the expression in the biological species. As a matter of course, DNA having thus converted codon is included in the scope of the present invention. For example, the genes having such an amino acid sequence as the base can be suitably synthesized by a method wherein an oligonucleotide synthesized with an automatic DNA-synthesizing machine is linked after the annealing.

As clearly described in a pamphlet of International Publication No. 02/20808, even when one or more amino acids are added or deleted in the protein of the lachrymatory factor-producing enzyme, it is possible to obtain the same enzymatic properties. Further, even when a part of the amino acid residues is replaced, it is also possible to obtain the same enzymatic properties. Such a modification of genes can be easily attained by using a gene site-specific variation-introducing kit available on the market, by inserting a synthetic gene or the like. In fact, it was proved that even when the amino acids are partially varied in green onion, shallot, echarote, leek or elephant garlic, the lachrymatory factor-producing enzymatic activity thereof can be obtained. Therefore, the gene of the lachrymatory factor-producing enzyme to be integrated into the vector can be its variant so far as the same enzymatic properties can be kept. Then the recombination expression vector is introduced into the host cells to obtain the transformant. The introduction of the recombination expression vector can be conducted by an ordinary method. The ordinary methods are various and they include, for example, competent cell method, protoplast method, calcium phosphate coprecipitation method, electroporation method, micro-injection method and liposome fusion method. Any suitable method may be selected depending on the host. Suitable examples of the hosts producing the lachrymatory factor-producing enzyme of the present invention are microorganisms such as Escherichia coli, Bacillus subtilis, yeast and *koji* molds, and cells such as cultured silkworm cells.

By culturing the transformant obtained as described above, the lachrymatory factor-producing enzyme can be produced in the culture mixture. This enzyme is isolated or purified by a well known method to stably obtain the lachrymatory factor-producing enzyme.

### (2) Means for repressing the expression of gene:

### (Nucleic acid molecule having a function of repressing the translation of mRNA of protein or polypeptide of lachrymatory factor-producing enzyme)

It was proved by the investigations of the present inventors that the lachrymatory factor-producing enzyme is an indispensable factor for forming the lachrymatory factor (J. P. KOKAI No. Hei 10-295373). Therefore, it is self-evident that when the action of this enzyme is repressed, the lachrymatory factor is not formed.

Various investigations were made for the purpose of repressing the formation of the lachrymatory factor. They include a cultivation method wherein the amount of sulfur-containing fertilizers is reduced for reducing the accumulation of S-1-propenyl-cysteine sulfoxide (PeCSO) which is a substrate for alliinase and a method for inactivating alliinase for attaining the purpose. However, they cannot solve the problems while the quality of the product is kept high.

Thus, the method for repressing the steps ranging from the transcription to the translation of the gene encoding the lachrymatory factor-producing enzyme is very useful for the production of allium vegetables having a high quality and a repressed lachrymatory effect. This method cannot be performed unless the gene sequence of the enzyme is elucidated.

Various methods known in the art can be employed for repressing the expression of the gene of the lachrymatory factor-producing enzyme. The repression of the expression of gene includes the repression of the transcription of genes and the repression of the translation thereof into protein. For effectively repressing the expression of the genes, it is effective to repress the translation of mRNA of the lachrymatory factor-producing enzyme contained in allium vegetables.

Well-known the techniques of the repression for the above-described purpose include an antisense method wherein the full length or a part of mRNA of intrinsic lachrymatory factor-producing enzyme is hybridized to form double strand RNA so that genes can be introduced while the subsequent translation is repressed and also RNAi method wherein double strand RNA of all the sequences of the enzyme or a part thereof is formed to introduce the gene so as to decompose mRNA of the intrinsic lachrymatory factor-producing enzyme. Another effective method comprises utilizing a co-repression wherein a gene is introduced to over-express the full length or a part of the sense strand or an analogous sequence of the lachrymatory factor-producing enzyme and consequently, the expression of a gene homologous thereto is repressed.

Namely, all the nucleic acid molecules capable of eliminating the function of the intrinsic mRNA by the above-described mechanisms or the like are effective irrespective of the length thereof, number of the strands (single strand or double strand) or the hybridization with the genes of the lachrymatory factor-producing enzyme. The length of the nucleic acid molecules is at least 18 nucleotides, preferably at least 22 nucleotides. To say repeatedly, reasons why the design or the performance of such nucleic acid molecules has become possible are that the gene sequence of the lachrymatory factor-producing enzyme was elucidated and the design or the performance of them has become possible on the bases of the sequence.

The sequence of the sense or antisense nucleotides used in the present invention is preferably complementary to the whole or a part of the sequence of endogenous genes (or homologous genes) of a vegetable to be transformed. However, the complementation may be incomplete so far as the expression of the genes can be effectively repressed. For example, RNA transcribed from DNA somprising at least one of the DNA sequences of the present invention is preferably hybridized to RNA transcribed from the genes of the lachrymatory factor-producing enzyme, regulatory sequence at upstream side thereof and RNA transcribed from the DNA sequence between them. This RNA may be of either single strand or double strand.

### (Test of the effect of nucleic acid molecules inhibiting the translation of intrinsic mRNA)

For examining whether a nucleic acid molecule repressed the translation of mRNA of the intrinsic lachrymatory factor-producing enzyme or not, it is effective to determine the lachrymatory factor-producing enzymatic activity of a vegetable tissue into which genes were introduced so as to translate the nucleic acid molecules into RNA or to determine the quantity of protein in the enzyme so as to directly confirm the effect. The fact that the lachrymatory factor-producing enzymatic activity is reduced or the amount of the protein in the enzyme is reduced indicates that the translation of intrinsic mRNA is repressed by the introduced nucleic acid molecules. The effectiveness of the introduced nucleic acid molecules can be judged from those results.

For example, the lachrymatory factor-producing enzymatic activity is determined by adding an extract of a vegetable tissue to be tested to the reaction system of alliinase extracted from garlic and free from this enzyme and PeCSO which is the substrate of alliinase, and determining the generated lachrymatory factor (LF) by HPLC or the like. More concretely, the fact whether a transformed vegetable has the lachrymatory factor-producing enzymatic activity or not can be confirmed by a method described in International Patent Application PCT/JP01/07465 as will be shown in Examples given below.

The fact that the amount of the protein in the lachrymatory factor-producing enzyme is reduced can be judged by western blotting method wherein an antibody of this enzyme prepared by using this enzyme as the antigen is used. Namely, this judgment can be conducted by the ordinary western blotting method wherein a fraction extracted from a vegetable tissue to be tested is fractionated by SDS-PAGE (SDS-polyacrylamide electrophoresis) and, after the blotting with PVDF membrane, the protein is selectively detected with the lachrymatory factor-producing enzyme antibody. The standard protein of the lachrymatory factor-producing enzyme used herein can be prepared by extracting it from various allium vegetables and purifying the extract. It is also possible to use a recombinant lachrymatory factor-producing enzyme obtained by the expression from DNA sequence of the enzyme with E. coli or the like. The determination method of the enzymatic activity and also the determination method of the amount of the protein in the lachrymatory factor-producing enzyme are not limited to the ordinary methods described herein but any method can be employed.

### (2-1) Production of vegetables which do not form lachrymatory factor by antisense RNA

The expression of the genes of a lachrymatory factor-producing enzyme can be repressed by introducing DNA having a reversed complementary strand sequence of the gene of the lachrymatory factor-producing enzyme of the present invention into an allium vegetable to express an antisense RNA in the vegetable. Because the antisense RNA has a base sequence complementary to mRNA of the gene of the lachrymatory factor-producing enzyme, the synthesis of the protein of the lachrymatory factor-producing enzyme (the final product) is repressed by forming a base pair with this mRNA. The antisense RNA usable in the present invention is an oligonucleotide capable of specifically hybridizing with mRNA translated into the lachrymatory factor-producing enzyme.

An antisense RNA sequence can be designed on the basis of a base sequence of alignment data of the above-described base sequence excluding a region having a low conservativeness. Because the base sequences of the genes of the lachrymatory factor-producing enzyme are inclined to be conserved in the whole allium vegetables excepting a region of a low conservativeness, the diversion between different vegetables is possible. Namely, for example, an antisense RNA designed from the reversed complementary strand of the gene sequence of the lachrymatory factor-producing enzyme of green onion can be expresses in onion.

The target site of antisense RNA is various depending on the gene. There is no consensus on the site. However, generally, ATG start site might be the target site. Further, recently, several kinds of computerized analysis software (such as HYB simulator) for designing the target site and antisense RNA are available on the market. It is possible to design the antisense RNA by using them. Preferably, the length of antisense RNA is at least 18 to 23 mer and GC content is at least 50 %.

In addition to the antisense RNA method, a method wherein sense strands are integrated and RNAi (RNA interference) method can also be employed. Although the sequence of the sense and antisense nucleotides is preferably complementary to that of the intrinsic genes (or homologous genes) or a part thereof of a vegetable to be transformed, the complete complementarity is not required so far as the effective repression of the expression of the genes is possible.

An example of a method for functioning of the above-described antisense RNA in a vegetable includes a method in which cDNA is integrated in the opposite direction at a downstream side of a promoter expressed with an eucaryote and then it is introduced into host cells to synthesis the antisense RNA.

Methods for introducing extrinsic genes include a transformation method with Agrobacterium and a transformation method by the direct introduction.

In the vegetables in which the expression of the genes of the lachrymatory factor-producing enzyme is repressed, thiosulfinates is produced in an increased amount because 1-propenylsulfenic acid is not converted into the lachrymatory factor. It was confirmed *in vivo* that thiosulfinates which are main smelling components of onion have antiasthmatic effects. *In vitro,* the thiosulfinates inhibit cyclooxygenase and 5-lipooxygenase (Wagner, H., Dorsch, W., Bayer, T., Breu, W. & Willer, F. Antiasthmatic effects of onions: inhibition of 5-lipoxygenase and cyclooxygenase in vitro by thiosulfinates and "Cepaenes". Prost Leuk. Essential Fatty Acids, 39, 59-62 (1990)). In addition, the thiosulfinates are converted into disulfides and trisulfides which have an effect of reducing lipids in the blood (Adamu, I., Joseph, P. K. & Augusti, K. T. Hypolipidemic action of onion and garlic unsaturated oils in sucrose fed rats over a two-month period. Experientia 38, 899-901 (1982)) and an effect of inhibiting the platelet aggregation (Ariga, T., Oshiba, S. & Tamada, T. Platelet aggregation repressor in garlic. Lancet 1, 150-151 (1981) and Makheja, A. N. & Bailey, J. M. Antiplatelet constituents of garlic and onion. Agents Actions 29, 360-363 (1990)), respectively. Thus, it is possible to produce transformed vegetables capable of producing an increased amount of the above-described thiosulfinates and the reaction products of them.

### (3) Mass production of protein of lachrymatory factor-producing enzyme

The plasmids integrating cDNA include, for example, pBR322 (Gene 2, 95 (1977)) and pBR325 (Gene 4, 121 (1978)) from E. coli and pUB110 from Bacillus subtilis (Biochemical and Biophysical Research Communication, 112, 678 (1983)). Any other plasmids can be used so far as they can be replicated and retained in the host. An ordinary method for integrating cDNA into the plasmids comprises preparing a liquid mixture of a vector and an insert in a molar ratio of 1:1 to 1:10 and treating it with T4 ligase (a separate volume of *Saibo Kogaku*, Biological Experiment illustrated, (2) The Fundamentals of Gene Analysis, p. 78, Shujun-sha). The plasmid thus obtained is introduced into a suitable host such as a bacterium of Escherichia or Bacillus.

Bacteria of Escherichia include, for example, Escherichia coli (Proc. Natl. Acad. Sci. U. S. A. 60, 160 (1968). Bacteria of Bacillus include, for example, Bacillus subtilis MI 114 (Gene, 24, 255 (1983)). Methods for the transformation include, for example, calcium chloride method (Biochemical Biophysical Research Communication, 49, 1568 (1972)). The intended clone is selected from thus obtained transformants by a known method such as a colony hybridization method (Gene 10, 63 (1980)) and DNA sequencing method (Proceeding of National Academy of Science, 74, 560 (1977)). Thus, microorganisms having a vector having DNA containing a base sequence encoding the cloned lachrymatory factor-producing enzyme are obtained.

Then the plasmid is isolated from the microorganism. The isolation method is, for example, an alkali method (Nucleic Acids Research), 1513 (1979)). The plasmid containing the base sequence encoding the cloned lachrymatory factor-producing enzyme is kept as it is or, if desired, it is cleaved with a restriction enzyme. The cloned genes can be linked at a downstream side of the promoter in the vector suitable for the expression to obtain an expression vector.

The vectors include, for example, Escherichia coli plasmid (such as pBR322), Bacillus subtilis plasmid (such as pUB110), Yeast plasmid (such as pSH19) and Bacteriophages such as λ phage, and animal viruses such as retrovirus and vaccinia virus. The gene may have ATG as a translation initiation codon at the 5' terminal thereof and also TAA, TGA or TAG as the translation termination codon at the 3' terminal thereof. When 5' terminal of a gene encoding a known protein is linked with 3' terminal thereof to express a fused protein, the translation initiation codon is not necessarily required. Further, for expressing the gene, a promoter is linked at an upstream side thereof. The promoter used in the present invention is not particularly limited so far as it is suitable for the host in the expression of the gene. When the host is a microorganism of the genus Escherichia in the transformation, trp promoter, lac promoter, recA promoter, etc. are preferred. When the host is a microorganism of the genus Bacillus, preferred promoters are SPO1 promoter, SPO2 promoter, penP promoter, etc. When the host is yeast, preferred promoters are PHO5 promoter, PGK promoter, GAP promoter, etc. It is particularly preferred that the host is a microorganism of the genus Escherichia and the promoter is lac promoter. When the host is an animal cell, promoters are, for example, SV40 promoter, retrovirus promoter, etc. In this case, SV40 promoter is particularly preferred.

The transformant is produced by using the vector containing thus constructed DNA. The hosts are, for example, microorganisms of the genus Escherichia or Bacillus, yeast and animal cells. Examples of the microorganisms of the genus Escherichia or Bacillus are those described above. The yeast is, for example, Saccharomyces cerevisiae AH22R. The animal cells are, for example, monkey cells COS-7, Vero and Chinese hamster cells CHO. Thus, the transformants transformed with the DNA-containing vector are obtained.

In an example, when a transformant obtained by using a bacterium of the genus Escherichia or Bacillus as the host is to be cultured, a liquid culture medium is suitable. The culture medium contains carbon sources, nitrogen sources, inorganic substances, etc. required for the growth of the transformant.

For culturing microorganisms of the genus Escherichia, preferred medium is, for example, LB medium or SOC medium (a separate volume of *Saibo Kogaku,* Bio-illustrated, 1. The Beginning of Molecular Biology Experiments, pp. 98-99, Shujun-sha). If necessary, a chemical such as isopropyl-1-thio-β-D-galactoside (IPTG) can be added to the medium for increasing the efficiency of the promoter. When the host is a microorganism of the genus Escherichia, the culture is usually conducted at 15 to 43°C for 3 to 24 hours, if necessary under stirring and aeration. When the host is a microorganism of the genus Bacillus, the culture is usually conducted at 30 to 40°C for about 6 to 24 hours, if necessary, under stirring and aeration. When the host is yeast, the transformant is cultured in, for example, Barkholder minimal medium (Proceeding of National Academy of Science 77, 4505 (1980)). pH of the medium is preferably controlled at about 5 to 8. The culture is usually conducted at 20 to 35°C for about 24 to 72 hours, if necessary, under aeration and stirring. When the host of a transformant to be cultured is animal cells, the medium is, for example, MEN medium containing about 5 to 20 % of fetal calf serum [Science 122, 501 (1952) DMEM medium (Virology) 8, 396 (1959)]. pH is preferably about 6 to 8. The culture is usually conducted at about 30 to 40°C for about 15 to 60 hours. If necessary, carbon dioxide concentration can be increased.

The protein of the lachrymatory factor-producing enzyme can be separated from the above-described culture mixture and purified by, for example, the following method: the protein of the lachrymatory factor-producing enzyme is extracted from the cultured microbes or cells by collecting the microbes or cells by a well-known method after the culture, suspending the microbes or cells in a buffer solution containing a protein-denaturalizing agent such as guanidine hydrochloride, breaking the microbes or cells with ultrasonic waves or a lysozyme and/or by freeze-thawing, and collecting the protein of lachrymatory factor-producing enzyme by the centrifugation. The protein of the lachrymatory factor-producing enzyme in the supernatant can be purified by a suitable combination of well-known separation and purification methods. Well-known methods for the separation and purification are, for example, a method wherein a difference in the solubility is employed such as salting out or solvent precipitation method, a method wherein a difference in the molecular weight is employed such as dialysis method or gel filtration method, a method wherein a difference in the charge is employed such as ion exchange chromatography, a method wherein a specific affinity is employed such as affinity chromatography, and a method wherein a difference in the hydrophobicity is employed such as reversed phase high-speed liquid chromatography.

Thus, the genes of the lachrymatory factor-producing enzyme of the present invention are widely usable as genetic engineering tools in the screening, the production of enzymes and protein, the production of transformed vegetables and the like. The base sequences of the genes of the lachrymatory factor-producing enzyme of the present invention are highly analogous to each other in allium vegetables. Therefore, the use of DNA from, for example, green onion is not limited to the technique for green onion. The genes of a vegetable can be used for another vegetable.

### Brief Description of the Drawings:

Fig. 1 shows the homology in the base sequences of SEQ ID NOS. 1, 3, 5, 7, 9, 11, 13 and 15.
Fig. 2 shows the homology in the estimated amino acid sequences of SEQ ID NOS. 2, 4, 6, 8, 10, 12, 14 and 16.
Fig. 3A shows the alignment data of the base sequences of SEQ ID NOS. 1, 3, 5, 7, 9, 11,13 and 15.
Fig. 3B shows the alignment data of the base sequences of SEQ ID NOS. 1, 3, 5, 7, 9, 11, 13 and 15 (continuation from Fig. 3A).
Fig. 3C shows the alignment data of the base sequences of SEQ ID NOS. 1, 3, 5, 7, 9, 11, 13 and 15 (continuation from Fig. 3B).
Fig. 4A shows the alignment data of the amino acid sequences of SEQ ID NOS. 2, 4, 6, 8, 10, 12, 14 and 16.
Fig. 4B shows the alignment data of the amino acid sequences of SEQ ID NOS. 2, 4, 6, 8, 10, 12, 14 and 16 (continuation from Fig. 4A).
Fig. 5 shows the sequence in E2/uni/f/298 region and sequences of 10 bases before and after the region corresponding to a sequence list of Universal primer E2/uni/f/298 sequence.
Fig. 6 shows the process for producing cDNA of E2-AF, E3-AC and E2-AA and subcloning them.
Fig. 7 shows processes for constructing expression plasmid and for producing the transformant.

### Best Mode for Carrying out the Invention:

### Example 1 (Genes of lachrymatory factor-producing enzyme from green onion)

### (1) Extraction of whole RNA

Fresh green onion (A. fistulosum L.) was instantaneously frozen with liquid nitrogen and then crushed with a mallet. The crushed frozen material was placed in a dry-sterilized mortar and then pulverized with a pestle. 100 mg of the pulverized frozen material was treated according to a protocol of RNeasy Plant Mini Kit (QIAGEN Cat. no. 74903) to obtain the whole RNA.

The yield of the obtained whole RNA was 13 µg.

### (2) Synthesis of cDNA

1^{st} strand cDNA was synthesized from the obtained whole RNA by the reverse transcription by using oligo(dT) (SEQ ID NO. 30) having an adopter sequence at the 5' terminal as the primer, according to the protocol of the kit, with Ready-To-Go T-Primed First-Strand Kit (Amercham Biosciences code no. 27-9263-01).

### (3) Amplification of 3' side region of corresponding cDNA by 3' RACE method

3' RACE experiments were conducted by using a composition shown in Table 1 for analyzing the base sequence in 3' region of the intended gene (cDNA). In the structure of the 1^{st} strand cDNA obtained in (2), adaptor sequence is at 5' terminal. Therefore, for the amplification of only intended cDNA from the 1^{st} strand cDNA by PCR, PCR was conducted by using ACE02f primer designed from the sequence in 3' side region of gene of lachrymatory factor-producing enzyme (SEQ ID NO. 11) of onion and NotI primer complementary to the adaptor sequence.

Sense (ACE02f): 5'－TGG AGG GTC CTG AGC ACA AG-3' (SEQ ID NO.
17)

Antisense (NotI): 5'-TGG AAG AAT TCG CGG CCG CAG-3' (SEQ ID NO.
18)

**Table 1**

| | Final conc. | µl |
|---|---|---|
| dH₂O | ― | 13.875 |
| 10x PCR buffer II (-MgCl₂) | X 1 | 2.5 |
| dNTP mixture | 0.2 mM | 2.5 |
| ACE02f (25 µM) | 0.5 µM | 0.5 |
| NotI (25 µM) | 1.5 µM | 1.5 |
| 25 mM MgCl₂ | 1.5 mM | 1.5 |
| Ampli Taq Gold (5U/µl) | 0.625 U | 0.125 |
| Template DNA | 0.2 to 20 ng/ µl | 2.5 |
| Total amount of liquid | ― | 25 |

PCR conditions were as follows: $\text{(1) 94°C, 9 minutes → (2) 94°C, 1 minute → (3) 54°C, 1 minute → (4)} \text{72°C, 1 minute → (5) 72°C, 5 minutes → (6) 4°C, retention}$

(1) to (4) were repeated 45 times.

After PCR, the reaction mixture was subjected to 2 % agarose gel electrophoresis (detection with ethidium bromide) to judge the presence or absence of PCR product of the intended size.

### (4) Amplification of 5' side region of corresponding cDNA by 5' RACE method

For analyzing the base sequence in 5 region of the intended gene (cDNA), 5' RACE experiments were conducted by using a composition shown in Table 2. These experiments were conducted according to 5' RACE System for Amplification of cDNA Ends, Version 2.0 (Invirtogen life technologies Cat. no. 18374-058).

1^{st} strand cDNA obtained in (2) was treated with RNaseH to obtain single strand cDNA. Then a nucleotide homopolymer (dC polymer) was added to 3' terminal thereof with terminal deoxynucleotide transferase (hereinafter referred to as "TdT"). PCR was conducted by using an adopter primer (AAP) having a sequence complementary to polyC sequence at 3' terminal and a primer (ACE02r) designed from the sequence in 5' side region of genes of lachrymatory factor-producing enzyme of onion with PolyC-added cDNA as the template. Thus, only the intended cDNA was amplified in the 1^{st} strand cDNA added with dC polymer at 5' end.

Sense (AAP): 5'-GGC CAC GCG TCG ACT AGT ACG GGI IGG GII GGG
IIG-3' (SEQ ID NO. 19)

Antisense (ACE02r): 5'-CTC TTC GAT TTT CTG ACC TAT CTC AGT
AGC-3' (SEQ ID NO. 20)

**Table 2**

| | Final conc. | µl |
|---|---|---|
| dH₂O | ― | 32.7 |
| 10x PCR buffer II (-MgCl₂) | X 1 | 5 |
| dNTP mixture (10 mM) | 0.2 mM | 1 |
| ACE02r (25 µM) | 0.4 µM | 0.8 |
| AAP (10 µM) | 0.4 µM | 2 |
| 25 mM MgCl₂ | 1.5 mM | 3 |
| Ampli Taq Gold (5U/µl) | 2.5 U | 0.5 |
| Template DNA | 0.2 to 20 ng/µl | 5 |
| Total amount of liquid | ― | 50 |

PCR conditions were as follows: $\text{(1) 95°C, 10 minutes → (2) 94°C, 1 minute → (3) 55°C, 1 minute →} \text{(4) 72°C, 1 minute → (5) 72°C, 7 minutes → (6) 4°C, retention}$

(2) to (4) were repeated 40 times.

After PCR, the reaction mixture was subjected to 2 % agarose gel electrophoresis (detection with ethidium bromide) to judge the presence or absence of PCR product of the intended size.

### (5) Analysis of base sequence of amplification product obtained by 3'RACE method or 5' RACE method, determination of the full length sequence and estimation of amino acid sequence

The obtained 3'RACE product or 5'RACE product was directly sequenced for analyzing the base sequence. The base sequence of the 3'RACE product and the base sequence of the 5'RACE product were assembled to determine the full length sequence of cDNA (shown by SEQ ID NO. 1) of the gene of the lachrymatory factor-producing enzyme in the sample. The open reading frame (ORF) was determined from the full length sequence of cDNA, and the amino acid sequence corresponding to each codon was estimated (shown by SEQ ID NO. 2).

### (6) Determination of base sequence of gene of the lachrymatory factor-producing enzyme of green onion by PCR amplification in ORF region and TA cloning of the product thereof

From the results of the analysis of the base sequence in (5) described above, the presence of cDNA corresponding to SEQ ID NO. 1, but in which adenine (A) at 244 in cDNA is replaced with guanine (G), was expected (adenine (A) was detected at a high peak and guanine (G) was detected at a low peak).

According to the estimated ORF sequence, the forward primer E2-AF-F (ATGGAGCTAAATCCTGGTGCGC (sequence No. 21) from the initiation codon (ATG) was synthesized.

PCR was conducted by using the synthesized primer E2-AF-F and NotI primer and also green onion cDNA as the template. The obtained amplification product was inserted into pGEM-T Easy Vector (a product of Promegu Co.) and then introduced into E. coli (XL1-Blue MRF') and the base sequence was analyzed. As a result, the presence of cDNA in which guanine (G) was No. 244 in SEQ ID NO. 1 was recognized. The sequence thus determined is shown in SEQ ID NO. 3. From the sequence, the amino acid sequence corresponding to each codon was estimated (shown in SEQ ID NO. 4).

### Example 2 (The full length sequence of cDNA of gene of lachrymatory factor-producing enzyme from shallot)

Fresh shallot (A. chinense L.) was used as the extraction material. The same methods as those in Example 1 were repeated for the extraction of whole RNA, synthesis of cDNA, amplification of 3' side region of the corresponding cDNA by 3'RACE method, amplification of 5' side region of the corresponding cDNA by 5'RACE method, analysis of the base sequence and the estimation of the amino acid sequence. The full length sequence of cDNA of the gene of lachrymatory factor-producing enzyme from shallot is shown in SEQ ID NO. 5, and the amino acid sequence corresponding to the codon is shown in SEQ ID NO. 6.

### Example 3 (The full length sequence of cDNA of gene of lachrymatory factor-producing enzyme from echarote)

Fresh echarote (A. chinense L.) was used as the extraction material. The same methods as those in Example 1 were repeated for the extraction of whole RNA, synthesis of cDNA, amplification of 5' side region of the corresponding cDNA by 5'RACE method, analysis of the base sequence and the estimation of the amino acid sequence.

However, in the amplification of 3' side region of the corresponding cDNA by 3'RACE method, nested PCR method was employed and PCR was conducted twice. In the first PCR, the sense primer in Example 1 was changed from ACE02f to E2-1-N (GGIGCI(A/C)GIAA(A/G)TGG (SEQ ID NO. 23)) and the PCR conditions (3) were changed from "54°C, 1 minute" to --43°C, 1 minute--. The second PCR was performed by using the obtained amplification product as the template. In the second PCR, PCR conditions and the primer used were the same as those in the amplification in 3' side region of the cDNA in 3' RACE method shown in Example 1.

The analyzed full length sequence of cDNA of the gene of lachrymatory factor-producing enzyme from echarote is shown in SEQ ID NO. 7, and the amino acid sequence corresponding to the codon is shown in SEQ ID NO. 8.

### Example 4 (cDNA sequence of gene of lachrymatory factor-producing enzyme from leek)

Fresh leek (A. ampeloprasum L.) was used as the extraction material. The same methods as those in Example 1 were repeated for the extraction of whole RNA, synthesis of cDNA, analysis of the base sequence and the determination of the amino acid sequence.

However, in the amplification of 3' side region of the cDNA by 3'RACE method, the amplification product could not be obtained not only when ACE02f (the sense primer of Example 1) was used but also when another sense primer designed on the basis of only the sequence of gene of lachrymatory factor-producing enzyme of onion was used. Under such circumstances, a region in which the sequence is conserved in all of the three kinds of genes of the lachrymatory factor-producing enzyme in onion, shallot and green onion was found (Fig. 5), and E2/uni/f/298 (GAATTTTGGGCCAAGGAGAAGCTGG (SEQ ID NO. 25) designed from the sequence thereof was used as the sense primer. Thus, the amplification in 3' side region of the cDNA was succeeded for the first time.

The results that the amplification product could not be obtained by using the primer designed from the sequence of onion were also obtained in 5'RACE. Therefore, E2/r/580-Le (CACACAGCATCACAAATTGAC (SEQ ID NO. 28)) was designed on the basis of the base sequence of leek 3' RACE product obtained by using E2/uni/f/298 as the sense primer and it was used as the antisense primer in 5'RACE method. Thus, the amplification in 5' side region of the cDNA was succeeded.

The obtained 3'RACE product or 5'RACE product was directly sequenced for analyzing the base sequence. The base sequence of the 3'RACE product and the base sequence of the 5'RACE product were assembled to estimate the full length sequence of cDNA of the gene of the lachrymatory factor-producing enzyme from leek. However, it was found from the results of the base sequence analysis that leek has two or more cDNA of gene of the lachrymatory factor-producing enzyme which have different base sequences. Thus, cDNA sequence of leek could not be determined by only RACE method.

Under those conditions, forward primer E2-AP-F2 (ATGGCGCAAAATCCTGGTGTGC (SE ID NO. 29)) was prepared from ATG the closest to 5'terminal of the full length sequence of cDNA estimated from the RACE results.

PCR was conducted by using the obtained primer E2-AP-F2 and NotI primer and also leek cDNA as the template. The obtained amplification product was introduced into pGEM-T Easy Vector and then introduced into E. coli (XL1-Blue MRF') and the base sequence was analyzed. As a result, two kinds of cDNA sequences were determined. The two kinds of the sequences of the leek thus determined are shown in SEQ ID NOS. 9 and 13. From each of the sequences, the amino acid sequence corresponding to each codon was estimated (shown in SEQ ID NOS. 10 and 14).

### Example 5 (The full length sequence of cDNA of gene of lachrymatory factor-producing enzyme from elephant garlic)

Fresh elephant garlic (A. ampeloprasum L.) was used as the extraction material. The same methods as those in Example 1 were repeated for the extraction of whole RNA, synthesis of cDNA, analysis of the base sequence and the estimation of the amino acid sequence.

However, in the amplification of 3' side region of the cDNA by 3' RACE method, the sense primer in Example 1 was altered from ACE02f to E2/uni/f/298 (GAATTTTGGGCCAAGGAGAAGCTGG (SEQ ID NO. 25)) designed by finding a sequence common to all the vegetables in the 3' side region of the genes of the lachrymatory factor-producing enzymes of other allium vegetables (onion, shallot and green onion).

In the amplification of 5' side region of the cDNA by 5' RACE method, the antisense primer in Example 1 was altered from ACE02f to a primer (E2-1-5R-1) (TCCTCGTACCCTGTAAAACACTCAG (SEQ ID NO. 26)) designed from the base sequence of onion.

The analyzed full length sequence of cDNA of the gene of lachrymatory factor-producing enzyme from the elephant garlic is shown in SEQ ID NO. 15, and the amino acid sequence corresponding to the codon is shown in SEQ ID NO. 16.

### Example 6 (Method for the preparation of protein)

### (1) Construction of expression plasmid

### (Shallot, echarote and elephant garlic)

PCR reaction was carried out by using a forward primer designed on the basis of the 5' terminal of an open reading frame of a gene sequence of lachrymatory factor-producing enzyme of SEQ ID NO. 5, 7 or 15, a reverse primer complementary to anchor part attached to oligo-dT primer and cDNA from each of the allium vegetables as the template to obtain about 700 bp of the product. The allium vegetables used were shallot, echarote and elephant garlic. The forward primers were E2-AC-F (ATGGAGCAAAATTCTGGTACGC (SEQ ID NO. 22)) for shallot, E2-AA-F (ATGGAGCTAAATCCTGGTGCAC (SEQ ID NO. 24)) for echarote and E2-APEG-F (ATGATGACATATCCTGGAAATCG (SEQ ID NO. 27)) for elephant garlic. As the reverse primer, a primer complementary to the anchor part of oligo-dT primer was used in all the cases.

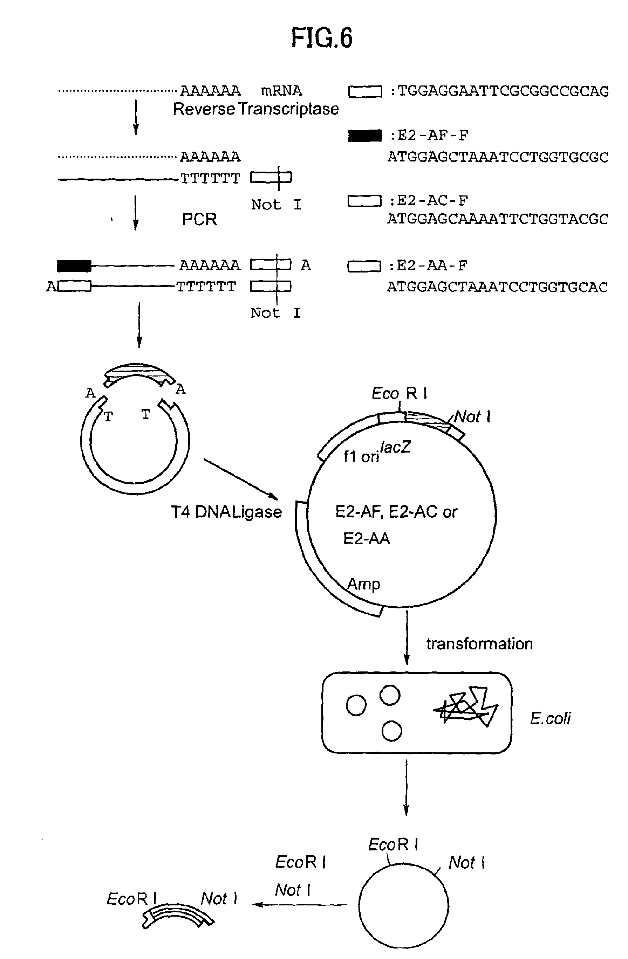

The obtained product was subcloned to obtain pGEM-T Easy Vector according to the above-described sequencing method and then it was introduced into E. coli (XL1-Blue) to analyze the base sequence thereof. The process for the subcloning is shown in Fig. 6.

From E. coli having pGEM-T-Easy Vector having the product integrated therein, E. coli having base sequence encoding a polypeptide of SEQ ID NO. 5, 7 or 15 [XL-1 Blue MRF'/pGEM-T-E2-AC (shallot), XL-1 Blue MRF'/pGEM-T-E2-AA (echarote) or XL-1 Blue MRF'/pGEM-T-E2-APEG (elephant garlic)] was obtained.

### (Green onion and leek)

For green onion and leek, E. coli having base sequence encoding a polypeptide of SEQ ID NO. 3, 9 or 13 prepared in Example 1 or 4 [XL-1 Blue MRF'/pGEM-T-E2-AF (green onion), XL-1 Blue MRF'/pGEM-T-E2-LK29A (leek 29A) or XL-1 Blue MRF'/pGEM-T-E2-LK58E (leek 58E)] was used.

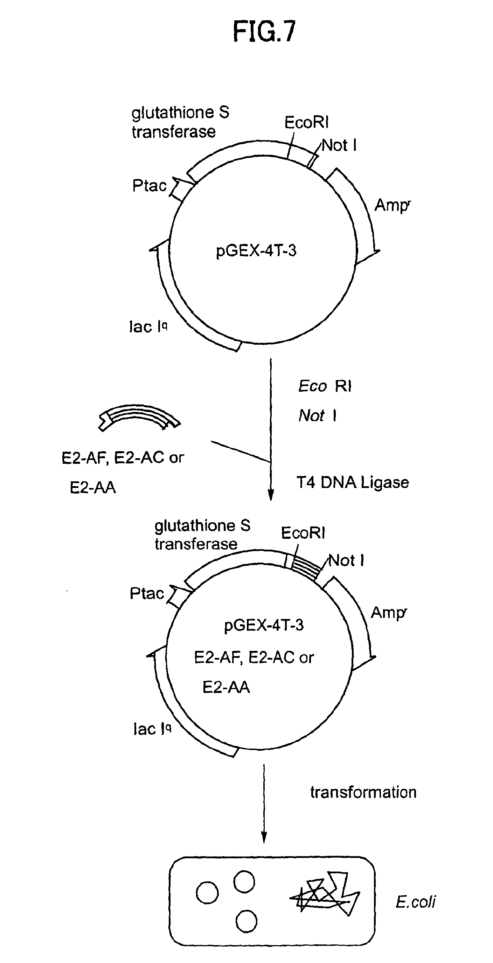

As the protein expression vector, pGEX-4T (Amercham Pharmacia) having a protease recognition site and multicloning site at a downstream side of the sequence of glutathione S transferase (GST) gene was used (Fig. 7).

A large fraction obtained by cleaving pGEX-4T with EcoRI (Takara Co., Ltd.) and NotI (Takara Co., Ltd.) was linked with about 700 bp fraction obtained by cleaving the above-described pGEM-T-E2-AF (green onion) with EcoRI and NotI to construct expression plasmid pGEX-4T-E2-AF (green onion).

A large fraction obtained by cleaving pGEX-4T with EcoRI (Takara Co., Ltd.) and NotI (Takara Co., Ltd.) was linked with about 700 bp fraction obtained by cleaving the above-described pGEM-T-E2-AC (shallot) with EcoRI and NotI to construct expression plasmid pGEX-4T-E2-AC (shallot).

A large fraction obtained by cleaving pGEX-4T with EcoRI (Takara Co., Ltd.) and NotI (Takara Co., Ltd.) was linked with about 700 bp fraction obtained by cleaving the above-described pGEM-T-E2-AA (echarote) with EcoRI and NotI to construct expression plasmid pGEX-4T-E2-AA (echarote).

A large fraction obtained by cleaving pGEX-4T with EcoRI (Takara Co., Ltd.) and NotI (Takara Co., Ltd.) was linked with about 700 bp fraction obtained by cleaving the above-described pGEM-T-E2-APEG (elephant garlic) with EcoRI and NotI to construct expression plasmid pGEX-4T-E2-APEG (elephant garlic).

A large fraction obtained by cleaving pGEX-4T with EcoRI (Takara Co., Ltd.) and NotI (Takara Co., Ltd.) was linked with about 700 bp fraction obtained by cleaving the above-described pGEM-T-E2-LK29A (leek: SEQ ID NO. 9) with EcoRI and NotI to construct expression plasmid pGEX-4T-E2-LK29A (leek: SEQ CO No. 9).

A large fraction obtained by cleaving pGEX-4T with EcoRI (Takara Co., Ltd.) and NotI (Takara Co., Ltd.) was linked with about 700 bp fraction obtained by cleaving the above-described pGEM-T-E2-LK58EA (leek: SEQ ID NO. 13) with EcoRI and NotI to construct expression plasmid pGEX-4T-E2-LK58E (leek: SEQ ID NO. 13).

### (2) Preparation of transformant of E. coli with expression plasmid and culture thereof:

The above-described pGEX-4T-E2-AF (green onion) was introduced into E. coli BL21-Gold (STRATAGENE Co.) by competent cell method to obtain transformant BL21-Gold/pGEX-4T-E2-AF (green onion) (Fig. 7).

The above-described pGEX-4T-E2-AC (shallot) was introduced into E. coli BL21-Gold (STRATAGENE Co.) in the same manner as that described above to obtain transformant BL21-Gold/pGEX-4T-E2-AC (shallot) (Fig. 7).

The above-described pGEX-4T-E2-AA (echarote) was introduced into E. coli BL21-Gold (STRATAGENE Co.) in the same manner as that described above to obtain transformant BL21-Gold/pGEX-4T-E2-AA (echarote) (Fig. 7).

The above-described pGEX-4T-E2-APEG (elephant garlic) was introduced into E. coli (BL21-GOLD (STRATAGENE Co.)) in the same manner as that described above to obtain transformant BL21-GOLD/pGEX-4T-E2-APEG (elephant garlic).

The above-described pGEX-4T-E2-LK29A (leek: SEQ ID No. 9) was introduced into E. coli (BL21-GOLD (STRATAGENE Co.)) in the same manner as that described above to obtain transformant BL21-GOLD/pGEX-4T-E2-LK29A (leek: SEQ ID NO. 9).

The above-described pGEX-4T-E2-LK58E (leek: SEQ ID NO. 13) was introduced into E. coli (BL21-GOLD (STRATAGENE Co.)) in the same manner as that described above to obtain transformant BL21-GOLD/pGEX-4T-E2-LK58E (leek: SEQ ID NO. 13).

The obtained transformant was cultured in LB medium containing 100 µg/ml of ampicillin under shaking at 37°C. Isopropyl-β-D-thiogalacto-pyranoside (IPTG) was added to the medium to induce the production. Thus, fused protein of GST and protein of each lachrymatory factor-producing enzyme (hereinafter fused protein will be referred to as follows: GST-E2-AF for green onion, GST-E2-AC for shallot, GST-E2-AA for echarote, GST-E2-APEG for elephant garlic, GST-E2-LK29A for leek (SEQ ID NO. 9) and GST-E2-LK58E for leek) (SEQ ID NO. 13) was accumulated in the microbes.

### (3) Isolation (purification) of protein

The transformants of all the vegetables were cultured as described above, and the microbes were collected by the centrifugation and then broken with ultrasonic waves. As for green onion, shallot and echarote, the supernatant recovered by the centrifugation was flown into Glutathione Sepharose 4 Fast Flow Column (Amercham Pharmacia) to adsorb GST fused protein on the column. The column was washed and the fused protein was eluted with an eluted buffer containing reduced glutathione to obtain three kinds of purified fused protein samples (GST-E2-AF, GST-E2-AC and GST-E2-AA).

Two kinds of fused protein samples were fed into HiTrap Desalting column (Amercham Pharmacia) to remove reduced glutathione. The sample was again adsorbed on Glutathione Sepharose 4 Fast Flow Column. The column was washed and then filled with a buffer containing thrombin. After the treatment with protease at room temperature for 2 hours, GST tag was separated from the fused protein. GST tag-free recombinant GST-E2-AF, GST-E2-AC and GST-E2-AA were eluted from the column. Benzamidine Sepharose 6B was added to the eluent, and they were mixed and then centrifuged to remove thrombin from the eluent, thereby obtaining three kinds of the recombinant samples (RC-E2-AF (green onion), RC-E2-AC (shallot) and RC-E2-AA (echarote)).

### (4) Activity of lachrymatory factor-producing enzyme of recombinant protein

The lachrymatory factor-producing enzymatic activity of 6 samples, i. e. fused protein samples GST-E2-AF, GST-E2-AC and GST-E2-AA, and also GST tag-free recombinant samples RC-E2-AF, RC-E2-AC and RC-E2-AA, was determined. As for elephant garlic and leek, the lachrymatory factor-producing enzymatic activity of supernatant liquid obtained by the centrifugation after the ultrasonic pulverization of the microbes in above-described step (3) (GST-E2-APEG/sup (elephant garlic), GST-E2-LK29A/sup (leek: SEQ ID NO. 9) and GST-E2-LK58E/sup (leek: SEQ ID NO. 13)) was determined.

The determination was conducted according to a method described in International Patent Application PCT/JP01/07465 as described below.

The recombinant protein sample obtained as described above was diluted with a diluting buffer (50 mM potassium phosphate buffer, pH 6.5). 40 µl of garlic alliinase (50 units/ml) and 20 µl of PeCSO solution (20 mg/ml) were added to 10 µl of the diluted sample. After carrying out the reaction at room temperature for 3 minutes, 1 µl of the reaction mixture was poured into HPLC to determine the amount of the lachrymatory factor thus obtained. For the analysis, ODS column (4.6 φ x 250 mm) (a product of Senshuu Kagaku Co.) was used. 30 % (v/v) acidic MeOH was used for the mobile phase, the flow rate was 0.6 ml/min, the column temperature was 35°C, and the detection was at 254 nm.

As a result, the lachrymatory factor-producing enzymatic activity was recognized in all of the fused protein samples. On the contrary, when a transformant (BL21-Gold/pGEX-4T-GST) obtained with expression plasmid pGEX-4T into which no gene of lachrymatory factor-producing enzyme was introduced was cultured and then the culture product was treated with glutathione Sepharose column, the lachrymatory factor-producing enzymatic activity was not detected. In a blank test wherein a phosphate buffer was used in place of the sample, the lachrymatory factor-producing enzymatic activity was not detected.

From the above-described results, it could be confirmed that even a fused protein formed by boning a large protein such as GST (molecular weight: about 27,000) to the N-terminal of the gene of the lachrymatory factor-producing enzyme has the lachrymatory factor-producing enzymatic activity.

The lachrymatory factor-producing enzymatic activity was recognized also in RC-E2-AF, RC-E2-AC and RC-E2-AA.

### Example 7 (Production of transformed vegetable)

### (1) Preparation of vector

A vector was prepared by linking any of the full length sequence of the gene of the lachrymatory factor-producing enzyme or a part thereof (preferably at least 18 bp) in a sense orientation of, an antisense orientation or both the orientations with a downstream of the regulatory region (promoter), linking a terminator with a downstream thereof and integrating the product into a plasmid. When the gene sequences of the lachrymatory factor-producing enzyme in both the sense orientation and the antisense orientation are connected with each other, then a terminator is connected with the downstream thereof and the product is integrated into the plasmid, it is preferred to insert "a spacer" (another base sequence) between the sense sequence and the antisense sequence. By this method, the stability of the vector in E. coli is improved. It is also preferred that the spacer contains "intron" (base sequence in non-translation region). In this case, the degree of the expression control in the individuals is increased and also the recovery of the expression-controlled individuals is increased. The following process can be conducted according to an ordinary gene cloning technique:
① A plasmid obtained by subcloning the gene of the lachrymatory factor-producing enzyme is introduced into E. coli (such as XL1-Blue) and proliferated. PCR is carried out by using the plasmid as the template to amplify the full length or a part of the sequence of the gene of the lachrymatory factor-producing enzyme. The amplified sequence is connected with a promoter to obtain an intended orientation. A terminator is added thereto and this sequence is integrated into the plasmid. When the sequences in both the sense orientation and the antisense orientation are contained therein, an intron-containing spacer is introduced between them.
   As the promoter, for example, 35S promoter of cauliflower mosaic virus is usable. Other promoters are also usable so far as the expression is possible in the vegetable cells. As the terminator, for example, nopaline synthase terminator is usable. Other terminators may also be used.
   Although ordinary plasmids such as pBI101 can be used as the intermediate vector plasmids for the integration of the genes, the plasmids are not limited thereto. When a plasmid having a suitable selection marker (a marker resistant to antibiotics such as hygromycin and kanamycin) is used for the integration of the gene, the selection of the transformant is facilitated.
② A gene inserted into the plasmid ① can be recombined with a plasmid in Agrobacterium by propagating the plasmid ① in E. coli (such as HB101 or SURE2) and triparentally mating this E. coli with E. coli having a helper plasmid (such as HB101 (pRK2013)) and Agrobacterium having helper Ti plasmid (such as pAL4404) (for example, Agrobacterium tumefaciens LBA4404 is preferred but other Agrobacterium bacteria such as EHA105 and EHA101 are also usable). In addition to the triparental mating with the helper plasmid, it is also possible to directly introduce the plasmid having the introduced gene sequence into Agrobacterium by the electroporation method.

### (2) Preparation of vegetable materials such as onion to be used for the recombination

① The vegetable materials are not limited so far as they have a re-differentiation faculty (faculty of regenerating the vegetable body). In Allium vegetables such as onion, a callus having the re-differentiation faculty derived from the body of the vegetable is preferably used. The organs of the allium vegetables such as onion, from which the callus is derived, are, for example, mature or premature embryos from seeds, germinated primary root from seeds, growth point of scaly leaf and basal plate of onion bulb.
② The composition of the culture medium in which the callus is derived is preferably the composition of MS medium usually used for the culture of vegetables but culture media having other compositions are also usable. An indispensable component of the medium for deriving the callus is auxin which is a phytohormone. The concentration of auxin is preferably 1 to 100 µM. Auxins preferred for deriving the callus include 4-FPA (4-fluorophenoxyacetic acid), Picrolam (4-amino-3,5,6-trichloro-2-pyridinecarboxylic acid) and 2,4-D (2,4-dichlorophenoxyacetic acid), etc. Other auxins are also usable.
③ The callus is cultured under conditions suitable for the culture. It is preferably cultured under irradiation with a fluorescent light of about 1000 to 3000 lux at 25°C. The derived callus can be maintained by the subculture. For using the callus keeping its re-differentiation faculty, it is preferred that the culture period for the derivation of the callus is shortened and that the number of times of the subculture is reduced. Concretely, period of the culture for the callus derivation is about 3 to 4 months and number of times of the subculture is 3 or below.
④ When onion or the like is used, the re-differentiation faculty of the callus significantly varies depending on the variety thereof. It is thus desirable to use a variety having a high re-differentiation faculty. Preferred varieties of onion are, for example, Sen-shyuu-chu-kodakaki, *kurena, momiji* and *tenju*.

### (3) Infection of callus with gene introduction vector

① The microbes of Agrobacterium having the gene introduction vector obtained in (1) were propagated and a callus is dipped in the microbe suspension. It is important in this step to add acetosyringone which is a compound required for the infection of monocotyledons with Agrobacterium. The concentration of acetosyringone is preferably 100 to 200 µM.
② After the cocultivation of the microbes and the callus for at least 3 days, preferably about 4 to 6 days, Agrobacterium is removed with an antibiotic such as claforan or carbenicillin.

### (4) Selection of transformant individuals from the infected callus

The callus is cultured and grown on a medium for a marker resistant to antibiotics such as hygromycin and kanamycin previously put into the vector and then it is re-differentiated. The living ones are the individuals succeeded in the transformation. As the composition of the culture medium used for the re-differentiation from the callus, the composition of MS medium usually used for the culture of vegetables can be used and culture media having other compositions are also usable. It is important to remove auxin from the re-differentiation medium.

### (5) Confirmation of transformant individuals

The introduction of the intended gene into the re-differentiated vegetable is confirmed by extracting DNA from the vegetable and examining it by Southern hybridization method (Hiroki Nakayama et al., Bio-experiment Illustrated ② *Idenshi Kaisetu no Kiso*, pages 137-151 (1995)).

### (6) Evaluation of characters of transformant individuals

The quantity of thiosulfinates contained in the re-differentiated vegetable and the reaction products thereof can be confirmed.

### Industrial Applicability

The lachrymatory factor-producing genes of the present invention have the following special effects: They are useful for designing antisense nucleotides required for repressing the expression level of the lachrymatory factor-producing enzyme and for increasing the amount of thiosulfinates and reaction products thereof having antiasthmatic effects. They are also capable of efficiently producing the lachrymatory factor-producing enzyme by the gene recombination technique. Lachrymatory factors useful for the treatment of dry eyes and the like can be thus efficiently produced.

## Claims

1. DNA encoding protein or a polypeptide capable of converting 1-propenylsulfenic acid into a lachrymatory factor.

2. DNA according to claim 1 which consists of a base sequence of SEQ ID NO. 1, 5, 7, 9, 13 or 15, or any of the following (a) to (c):
(a) DNA consisting of a base sequence of SEQ ID NO. 1, 5, 7, 9, 13 or 15 in which one or more of the bases are added, deleted or replaced,
(b) DNA capable of being hybridized with DNA consisting of a base sequence of SEQ ID NO. 1, 5, 7, 9, 13 or 15 under stringent conditions, and
(c) DNA consisting of a base sequence having a homology of at least 60 % with a base sequence of SEQ ID NO. 1, 5, 7, 9, 13 or 15.

3. A primer for using for isolating a gene of a lachrymatory factor-producing enzyme of SEQ ID NO. 25 of a vegetable of the genus allium.

4. A method for isolating a gene of a lachrymatory factor-producing enzyme of a vegetable of the genus allium with a primer of SEQ ID NO. 25.

5. A recombinant vector containing the DNA of claim 1 or 2.

6. A transformant containing a recombinant vector containing the DNA of claim 1 or 2.

7. A method for preparing protein or a polypeptide having lachrymatory factor-producing enzymatic activity by culturing host cells transformed with a recombinant vector containing the DNA of claim 1 or 2 and then separating protein or the polypeptide having lachrymatory factor-producing enzymatic activity produced in the medium or cells.

8. Protein or a polypeptide having lachrymatory factor-producing enzymatic activity, which contains any of the following (a) to (c):
(a) an amino acid sequence represented by SEQ ID NO. 2, 6, 8, 10, 14 or 16,
(b) an amino acid sequence represented by SEQ ID NO. 2, 6, 8, 10, 14 or 16 in which one or more of the amino acids are added, deleted or replaced, and
(c) an amino acid sequence having a homology of at least 65 % with an amino acid sequence of SEQ ID NO.2, 6, 8, 10, 14 or 16.

9. A nucleic acid molecule having a function of repressing the translation of mRNA on the protein or polypeptide of claim 8.
